# FASCICULE DE BREVET EUROPEEN

(11) **EP 4 507 639 B1**
(45) Date de publication et mention de la délivrance du brevet: **05.08.2026**
(21) Numéro de dépôt: 23725280.4
(22) Date de dépôt: 14.04.2023
(51) Int. Cl.: A61F 9/00

(54) **IMPLANT OPHTALMOLOGIQUE D'INTERPOSITION A EXCROISSANCE DE CONTACT**
OPHTHALMOLOGISCHES ZWISCHENPOSITIONIERUNGSIMPLANTAT MIT EINGRIFFSVORSPRUNG
OPHTHALMOLOGICAL INTERPOSITION IMPLANT WITH ENGAGEMENT PROTRUSION

(30) Priorité: 15.04.2022 FR 2203542
(43) Date de publication de la demande: 19.02.2025
(73) Titulaire: CILIATECH, 74370 Epagny Metz-Tessy (FR)
(72) Inventeur: BENOIT, Olivier, 74330 Epagny Metz-Tessy (FR); SOURDILLE, Philippe, 44350 GUERANDES (FR)
(74) Mandataire: Ipsilon
(86) Numéro de dépôt international: PCT/FR2023/050543
(87) Numéro de publication internationale: WO 2023/199007

(56) Documents cités:
- WO-A1-2016/156727
- US-A- 5 558 630
- US-A- 5 601 094
- US-A1- 2004 260 227
- US-A1- 2018 078 416
- US-B2- 7 207 965

## Description

La présente invention concerne un implant ophtalmologique d'interposition destiné à collecter l'humeur aqueuse depuis la chambre antérieure vers l'espace supra-ciliaire et supra-choroïdien pour abaisser durablement la pression intra oculaire (PIO).

La pression intra-oculaire résulte d'un équilibre entre la sécrétion par le corps ciliaire d'humeur aqueuse et son écoulement à travers les mailles du trabéculum cornéo-scléral par le canal de Schlemm et ses émonctoires jusqu'aux veines aqueuses et à la circulation générale. Une fraction de cet écoulement variable de 5 à 30% en fonction de l'âge se fait directement à travers le trabeculum uvéal entre la sclère et le corps ciliaire, c'est ce qu'on appelle l'écoulement uvéo-scléral. Les fibres longitudinales du muscle ciliaire, particulièrement au cours de l'accommodation, jouent un rôle de mise en tension des mailles trabéculaires, ce qui facilite l'écoulement uvéo-scléral de l'humeur aqueuse. C'est aussi au travers de ces fibres longitudinales du muscle ciliaire que s'écoule le flux uvéo-scléral.

Dans le glaucome l'écoulement de l'humeur aqueuse est diminué au niveau du trabéculum cornéo-scléral, ce qui entraine dans la majorité des cas une augmentation de la pression intra oculaire (PIO). L'abaissement de cette PIO est donc l'élément déterminant du traitement médical et/ou chirurgical du glaucome. Le traitement chirurgical repose sur deux possibilités : diminuer la production d'humeur aqueuse élaborée par le corps ciliaire (cyclo affaiblissement) ou augmenter l'écoulement d'humeur aqueuse en la dérivant. Cette dérivation est effectuée de différentes façons :
- en faisant communiquer directement la chambre antérieure et l'espace supra choroïdien (cyclodialyse et ses dérivés techniques), mais l'effet obtenu est le plus souvent transitoire et insuffisant. En désinsérant l'insertion du muscle ciliaire à l'éperon scléral cette technique d'implantation supprime le mécanisme physiologique de l'écoulement uvéo-scléral dans la zone chirurgicale. De plus la fibrose post-opératoire s'étend au-delà de cette zone. Il est par ailleurs connu que la partie de l'implant située dans la chambre antérieure peut toucher, même de manière intermittente ; l'endothélium cornéen, entrainant de ce fait un risque significatif d'œdème cornéen évolutif,
- en incisant, à partir de la chambre antérieure, le trabéculum jusqu'au canal de Schlemm pour court-circuiter l'obstacle trabéculaire. Cette intervention peut être complétée par la pose dans le canal d'un stent ouvert vers la chambre antérieure pour maintenir un accès direct permanent de l'humeur aqueuse au canal. Là aussi, les résultats sont souvent partiels et temporaires, et n'exonèrent pas de la continuation ou de la reprise du traitement médical.

La chirurgie filtrante reste un traitement de référence et cherche à dériver l'humeur aqueuse sous la conjonctive pour obtenir la baisse de pression nécessaire. Elle peut créer un orifice permanent de pleine épaisseur dans le trabéculum sous un volet scléral : la trabéculectomie. Une de ses variantes laisse en place la partie interne du trabéculum, ce qu'on appelle la chirurgie non perforante du trabéculum (sclérectomie profonde, viscocanalostomie).

Cependant, la chirurgie filtrante connait des complications liées à l'insuffisance de la filtration par fibrose de la bulle de filtration (la bulle de filtration est présente entre la sclère et la conjonctive qui s'en trouve soulevée) ou, à l'inverse, une filtration excessive qui peut être liée à l'utilisation per opératoire de mitomycine C.

Il est par ailleurs connu de l'état de l'art de nombreuses tentatives de normalisation de la PIO grâce à des implants, adossés aux procédures chirurgicales citées plus avant ou utilisés seuls. Or ces implants déforment tous plus ou moins l'anatomie de l'œil et/ou détournent/modifient les voies d'écoulement naturel. De plus, ces implants sont dessinés pour correspondre aux dimensions d'un œil standard ; ils ne s'adaptent pas aux variations d'anatomie présentes dans la nature.

Il est notamment connu du document WO 2016/156727 A1 d'implanter un implant intraoculaire permettant d'accroître et de rendre permanent l'effet hypotenseur de l'écoulement uvéo-scléral physiologique en interposant, entre la sclère et le corps ciliaire, un implant qui n'altère pas les structures anatomiques, avec ou sans intervention ajoutée, filtrante ou autre.

L'implant du document WO 2016/156727 A1 comprend un corps comprenant un bord antérieur destiné à se situer au plus près du trabéculum uvéal lorsque l'implant est disposé entre la sclère et le corps ciliaire. Ce bord antérieur est concave et forme une arête permettant un contact linéaire et continu entre le bord antérieur et le muscle ciliaire et son tendon au niveau de son insertion sur l'éperon scléral. Cette géométrie permet notamment un contact intime avec la zone d'écoulement du trabéculum uvéal.

Un inconvénient identifié avec ce type d'implants ophtalmologiques d'interposition ayant un contact linéaire et continu avec la zone d'écoulement du trabéculum uvéal, comme celui du document WO 2016/156727 A1, est que l'implant ophtalmologique bloque une partie du flux d'humeur aqueuse en raison du contact étroit entre les tissus et l'implant ophtalmologique. Un implant ophtalmologique d'interposition permanente selon le préambule de la revendication 1 est connu du document US7207965.

Un autre inconvénient avec ce type d'implants ophtalmologiques est qu'ils ne s'adaptent pas à la variabilité anatomique que l'on peut rencontrer chez les patients. En particulier, il a été observé que le diamètre de la chambre antérieure de l'œil peut varier chez les patients entre 10,75mm et 13,75mm. Ainsi, la performance de l'implant ophtalmologique peut dépendre de l'anatomie du patient.

Il existe donc un besoin pour un implant ophtalmologique ne présentant pas les inconvénients mentionnés ci-dessus. En particulier, il existe un besoin pour un implant ophtalmologique favorisant davantage l'écoulement de l'humeur aqueuse depuis le trabéculum uvéal tout en conservant un bon positionnement antérieur et en s'adaptant à différentes anatomies de patients.

Pour cela, l'invention propose un implant ophtalmologique d'interposition permanente entre la sclère et le tissu uvéal, caractérisé en ce que l'implant comprend un corps uvéocompatible, formé d'une seule partie, le corps comportant trois dimensions dans l'espace, à savoir une longueur et une largeur qui sont perpendiculaires entre elles et à une épaisseur, le corps de l'implant comprenant un premier bord dit antérieur destiné à être orienté en direction de la chambre antérieure d'un œil au contact de la racine du muscle ciliaire et un deuxième bord dit postérieur opposé au bord antérieur par rapport au corps. Etant donné la position souhaitée de l'implant, le bord antérieur de l'implant ne peut être en contact qu'avec la paroi postérieure de la racine du muscle ciliaire. La paroi antérieure de la racine du muscle ciliaire se trouve en chambre antérieure et en constitue une de ses limites. L'usage du terme « au contact de la racine du muscle ciliaire » tel qu'utilisé dans le présent document doit se comprendre comme « au contact de la paroi postérieure de la racine du muscle ciliaire ».

Le bord antérieur présente, suivant une vue en projection dans le plan contenant la largeur et la longueur, au moins une excroissance orientée vers l'extérieur du corps pour permettre au moins un contact local de ladite au moins une excroissance sur la racine du muscle ciliaire.

Une géométrie de l'implant induisant un contact local entre le bord antérieur et la racine du muscle ciliaire permet de libérer un espace entre le corps de l'implant et la racine du muscle ciliaire favorisant la libération de l'humeur aqueuse depuis le trabeculum uvéal et son écoulement vers le corps ciliaire et la sclère.

De plus, le contact local, qu'il soit unique ou multiple, permet au bord antérieur de pouvoir s'adapter à différentes anatomies du patient. En effet, il est plus aisé pour l'implant de se positionner contre la racine du muscle ciliaire malgré des diamètres différents de la chambre antérieure.

On entend par contact « local » le fait qu'une portion uniquement du bord antérieur est destinée à être en contact avec la racine du muscle ciliaire. Ainsi, le bord antérieur est conformé de telle sorte qu'il n'y ait pas de contact avec la racine du muscle ciliaire sur la totalité du bord antérieur.

L'invention concerne un implant ophtalmologique d'interposition permanente selon la revendication 1.

Les revendications dépendantes incluent les modes de réalisation de l'invention.

### Brève description des dessins

Les dessins annexés illustrent l'invention :
[Fig. 1] représente une vue générale très schématique montrant en vue de face une forme de réalisation possible d'un implant selon l'invention positionnés autour de la cornée d'un œil.
[Fig. 2] représente une vue détaillée d'un bord antérieur d'un implant selon l'invention comprenant une pluralité d'excroissances permettant un contact avec la racine du muscle ciliaire de l'œil.
[Fig. 3] représente une vue détaillée d'un bord antérieur d'un implant selon l'invention comprenant une pluralité d'excroissances formant des portions d'arête de contact s'étendant le long de trajectoires circulaires ayant des rayons de courbure différents.
[Fig. 4] représente une vue de côté d'un implant selon l'invention dont l'épaisseur du bord antérieur est amincie par rapport au corps de l'implant.
[Fig. 5] représente une vue de côté d'un implant selon l'invention dont l'épaisseur du bord antérieur est amincie par rapport au corps de l'implant, le bord antérieur formant également un amincissement local sur une face inférieure ou supérieure de l'implant.
[Fig. 6] représente un premier exemple de réalisation du bord antérieur d'un implant selon l'invention comprenant une excroissance.
[Fig. 7] représente un deuxième exemple de réalisation du bord antérieur d'un implant selon l'invention comprenant une excroissance.
[Fig. 8] représente un troisième exemple de réalisation du bord antérieur d'un implant selon l'invention comprenant deux excroissances.
[Fig. 9] représente un quatrième exemple de réalisation du bord antérieur d'un implant selon l'invention comprenant deux excroissances.
[Fig. 10] représente un cinquième exemple de réalisation du bord antérieur d'un implant selon l'invention comprenant deux excroissances.
[Fig. 11] représente un sixième exemple de réalisation du bord antérieur d'un implant selon l'invention comprenant deux excroissances.
[Fig. 12] représente un septième exemple de réalisation du bord antérieur d'un implant selon l'invention comprenant une pluralité d'excroissances.
[Fig. 13] représente un huitième exemple de réalisation du bord antérieur d'un implant selon l'invention comprenant une pluralité d'excroissances.
[Fig. 14] représente un neuvième exemple de réalisation du bord antérieur d'un implant selon l'invention comprenant une pluralité d'excroissances formant des portions d'arêtes de contact s'étendant le long de trajectoires circulaires de rayons de courbure différents.
[Fig. 15] représente de manière très schématique la mise en place d'un implant suivant une première méthode.
[Fig. 16] représente de manière très schématique la mise en place d'un implant suivant une deuxième méthode.
[Fig. 17] représente une vue de mise en situation schématisée dans un œil humain d'une forme générale simplifiée possible d'un implant selon l'invention interposé entre la sclère et le corps ciliaire.
[Fig. 18] représente une vue plus détaillée et agrandie de la structure de l'angle iridocornéen sans l'implant de la figure 17.
[Fig. 19] représente une vue de dessus d'un implant comprenant plusieurs excroissances et un premier exemple de réalisation d'un aiguillon destiné à être inséré dans la chambre antérieur d'un œil.
[Fig. 20] représente un implant comprenant un deuxième exemple de réalisation d'un aiguillon.
[Fig. 21] représente un implant comprenant un troisième exemple de réalisation d'un aiguillon.
[Fig. 22] représente un implant comprenant un quatrième exemple de réalisation d'un aiguillon.
[Fig. 23] représente une vue de côté d'un implant dont l'épaisseur de l'aiguillon est amincie par rapport au corps de l'implant.
[Fig. 24] représente une vue de dessus d'un implant dont la largeur de l'aiguillon diminue depuis une extrémité proximale solidaire du corps vers une extrémité distale pour faciliter l'insertion de l'aiguillon dans la chambre antérieure de l'œil.
[Fig. 25] représente une vue de côté d'un implant dont l'épaisseur de l'aiguillon est supérieure à l'épaisseur du corps de l'implant.
[Fig. 26] représente un implant comprenant deux aiguillons destinés à être insérés dans la chambre antérieure d'un œil.
[Fig. 27] représente de manière très schématique la mise en place d'un implant suivant une troisième méthode.
[Fig. 28] représente une vue de côté d'un aiguillon présentant une pointe distale de perforation présentant un bord biseauté pour permettre la perforation des tissus de la racine du muscle ciliaire.
[Fig. 29] représente une vue en perspective d'un premier exemple d'implant creux dont les faces supérieure et inférieure sont séparées par une cavité centrale.
[Fig. 30] représente une vue en perspective d'un deuxième exemple d'implant creux dont les faces supérieure et inférieure sont séparées par une cavité centrale.
[Fig. 31] représente une vue de côté de l'implant de la figure 30.
[Fig. 32] représente un troisième exemple d'implant creux dont les faces supérieure et inférieure sont séparées par une cavité centrale et qui présente un aiguillon avec une pointe distale de perforation biseautée.
[Fig. 33] représente une vue de côté de l'implant de la figure 32.

### Description de mode(s) de réalisation

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation. De plus, le terme « comprenant » n'exclut pas d'autres éléments ou étapes.

La présente invention est illustrée à l'aide des figures qui montrent un implant ophtalmologique d'interposition qui est destiné à collecter l'humeur aqueuse depuis la chambre antérieure vers l'espace supra-choroïdien pour abaisser durablement la pression intra oculaire (PIO).

Pour des raisons de clarté du présent exposé, l'implant ophtalmologique d'interposition pourra être appelé « implant » ci-après.

L'implant comprend un corps uvéocompatible, i.e. réalisé dans au moins un matériau qui est connu pour ses propriétés d'uvéocompatibilité. De telles propriétés procurent au corps une très faible adhérence aux tissus oculaires. En d'autres termes, ledit au moins un matériau n'est pas susceptible d'altérer les structures sus-jacentes et sous-jacentes par suite de la mise en contact du corps avec ces structures et de leurs mouvements répétés au cours du temps.

Le corps de l'implant comporte trois dimensions dans l'espace : une épaisseur, une longueur et une largeur qui sont perpendiculaires à l'épaisseur. Le ratio entre longueur et largeur peut être inférieur, égal ou supérieur à 1. A titre d'exemple, l'implant peut avoir pour dimensions (largeur, longueur, épaisseur) 4*5*0.1 mm, ou encore 3*7*0.8 mm, ou encore 6*3*0,6 mm. Par ailleurs, l'épaisseur de l'implant peut varier entre le bord antérieur et le bord postérieur, avec par exemple 0.15 mm au bord antérieur et 0.7 mm au bord postérieur. Cette variation peut être graduelle, aléatoire, sur la totalité du parcours antéro-postérieur et/ou locale.

L'implant est de préférence formé d'une seule partie en ce sens qu'il est formé une seule pièce monobloc. L'implant n'est donc pas un assemblage de plusieurs pièces fixées entre elles pour former le corps.

Le corps de l'implant est de préférence mince afin que, une fois interposé entre la sclère et le tissu uvéal, il ne déforme pas les tissus sus- et sous-jacents de manière inacceptable. Une déformation acceptable des tissus est une déformation qui n'altère pas la ou les fonctions de l'un et/ou de l'autre de ces tissus. L'épaisseur du corps 22 de l'implant 20 est de préférence comprise entre 50 et 1 000µm.

Le corps de l'implant comprend deux bords opposés qui sont éloignés l'un de l'autre suivant une des deux dimensions perpendiculaires à l'épaisseur. Les deux bords opposés comprennent un bord antérieur et un bord postérieur opposé au bord antérieur par rapport au corps.

Le bord antérieur est destiné à être orienté en direction de ou face à la chambre antérieure d'un œil et placé au contact de la paroi postérieure de la racine du muscle ciliaire,

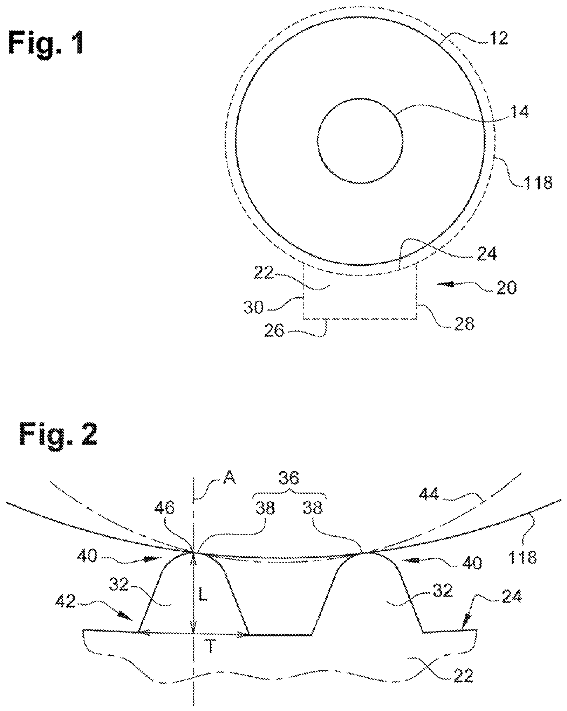

Comme représenté à la figure 1, un œil 10 est représenté en vue de face de manière très schématique par la cornée 12, la pupille 14 au centre et la racine du muscle ciliaire 118. Lorsque la racine du muscle ciliaire 118 est mentionnée dans le présente texte, il s'agit de paroi postérieure de la racine du muscle ciliaire.

Un implant 20 comprend un corps 22 ayant un bord antérieur 24 et un bord postérieur 26. Des bord latéraux 28 et 30 s'étendent de part et d'autre du corps entre les bords antérieur 24 et postérieur 26. La géométrie de l'implant sur la figure 1 est schématique. La figure 1 a pour objectif de définir le positionnement de l'implant 20 vis-à-vis de la racine du muscle ciliaire 118et pour identifier les bords antérieur 24, postérieur 26 et latéraux 28, 30. Les caractéristiques géométriques de l'implant 20 sont détaillées ci-après.

Le bord postérieur 26 est de préférence convexe. Un bord convexe décrit une courbe s'éloignant du corps 22 de l'implant 20 là où, inversement, un bord concave décrit une courbe rentrant ou se rapprochant du corps 22 de l'implant 20. De manière alternative, le bord postérieur 26 peut être rectiligne ou concave.

Selon un mode de réalisation possible, la convexité du bord postérieur 26 est prolongée par les bords latéraux 28, 30, i.e. sans angle formé entre les bords latéraux 28, 30 et le bord postérieur 26.

De manière générale (quelle que soit la forme de l'implant), le bord postérieur 26 doit être suffisamment éloigné du bord antérieur afin de procurer un effet d'écartement efficace. En pratique, le bord postérieur est de préférence éloigné du bord antérieur d'une distance d'au moins 1.0 mm.

De manière générale (quelle que soit la forme de l'implant), la longueur du bord postérieur 26 ne dépasse pas de plus de 50% la longueur du bord antérieur.

Les bords latéraux 28, 30 sont symétriques l'un par rapport à l'autre bien que, selon une variante non représentée, ils puissent être asymétriques l'un par rapport à l'autre.

Les bords latéraux 28, 30 peuvent être agencés de manière radiale (bords latéraux convergeant en un point fictif situé devant le bord antérieur), de manière évasée (bords latéraux convergeant en un point fictif situé devant le bord postérieur) ou bien encore parallèles entre eux.

Le corps 22 de l'implant 20 possède deux grandes faces opposées écartées l'une de l'autre suivant l'épaisseur du corps 22. Les deux grandes faces opposées comprennent une face supérieure et une face inférieure séparées par l'épaisseur. La face supérieure est destinée à être au contact de la sclère. La face inférieure est destinée à être au contact du corps ciliaire.

Le corps 22 de l'implant 20 peut présenter l'une des conformations suivantes dans une direction perpendiculaire à un plan défini par les deux dimensions de l'implant qui sont perpendiculaires à l'épaisseur :
- la face supérieure est plane et la face inférieure est concave,
- la face supérieure est plane et la face inférieure est convexe, et
- les faces supérieure et inférieure sont convexes
- les faces supérieure et inférieure sont planes

Selon un mode de réalisation, le corps 22 peut être creux. Ainsi, le corps 22 peut former une cavité centrale entre les faces supérieure et inférieure. La cavité centrale peut être ouverte sur l'extérieur de l'implant, par exemple au niveau d'un ou plusieurs bords parmi les bords latéraux et le bord postérieur. Cette cavité centrale est dépourvue en tout ou partie de matière, notamment le long de la distance antéro-postérieure.

Selon un mode de réalisation de l'implant, le corps est formé par une plaque repliée sur elle-même de manière à former la cavité centrale entre au moins deux portions de cette plaque se faisant face. Le corps peut ainsi être formé par un élément principalement bidimensionnel, ici une plaque, dont l'agencement, ici un pliage, permet de former un corps tridimensionnel dont l'épaisseur est supérieure à l'addition de l'épaisseur des deux portions de plaque se faisant face. Tout ou partie des parois de l'implant 20, à savoir les faces supérieure et inférieure, les bords latéraux ainsi que les bords antérieur et postérieur, peut être ajouré. En d'autres termes, ces parois peuvent être réalisées sous la forme d'un maillage faisant apparaître une alternance de matière et de trous.

Ce maillage peut être obtenu par enlèvement de matière ou bien par tissage de fils de matière. L'implant 20 peut être réalisé sous la forme d'une pluralité de parois principalement bidimensionnelles et tissées formant une enveloppe tridimensionnelle renfermant la cavité centrale. Un tel mode de réalisation utilisant des parois ajourées permet de favoriser la collecte et l'écoulement de l'humeur aqueuse. L'implant 20 peut comprendre une combinaison de parois ajourées et de parois pleines.

Le bord antérieur 24 présente au moins une excroissance 32 orientée vers l'extérieur du corps 22. Ladite au moins une excroissance 32 fait saillie hors du corps 20 suivant une vue en projection dans le plan contenant la largeur et la longueur, tel que visible en figure 1. Cette excroissance 32 permet au moins un contact local de ladite au moins une excroissance sur la racine du muscle ciliaire.

On entend par contact « local » le fait qu'une portion uniquement du bord antérieur 24 est destinée à être en contact avec la racine du muscle ciliaire. Ainsi, le bord antérieur 24 est conformé de telle sorte qu'il n'y ait pas de contact avec la racine du muscle ciliaire sur la totalité du bord antérieur 24.

Le bord antérieur 24 peut comprendre une seule excroissance 32 de manière à simplifier la géométrie du bord antérieur 24 tout en maximisant la collecte d'humeur aqueuse. Ladite excroissance 32 est de préférence disposée au niveau d'une partie centrale du bord antérieur 24 pour permettre un meilleur positionnement de l'implant 20 sur la racine du muscle ciliaire.

En référence à la figure 2, le bord antérieur 24 comprend une arête de contact 36 qui forme la zone de contact entre le bord antérieur 24 et la racine du muscle ciliaire 118. Chaque excroissance 32 parmi ladite au moins une excroissance 32 comprend une portion d'arête de contact 38 au niveau d'une extrémité distale 40. Lorsque le bord antérieur comprend une pluralité d'excroissance 32, l'arête de contact 36 est formée par la pluralité de portions d'arête de contact 38 portées par les excroissances 32.

Dans le cas d'une pluralité d'excroissance 32, ces dernières forment conjointement une arête de contact 36 discontinu ou local. En d'autres termes, le contact entre le bord antérieur 24 et la racine du muscle ciliaire est discontinu car réalisé par une pluralité de portion d'arête de contact 38. De plus, le contact peut être local en ce qu'il n'est pas réalisé par la totalité du bord antérieur 24 mais seulement par une partie de ce bord antérieur 24. De manière préférée, l'arête de contact 36 permet un contact discontinu et local en ce que le contact est réalisé sur une pluralité de zones de contact localisées.

Augmenter le nombre de portions d'arêtes de contact 38 permet une meilleure mise en position et stabilité de l'implant 20 contre la racine du muscle ciliaire 118 ce qui améliore la collecte d'humeur aqueuse.

Ladite au moins une excroissance 32 peut être configurée pour permettre au moins un contact ponctuel sur la racine du muscle ciliaire. Il convient de noter que les tissus du muscle ciliaire sont flexibles et qu'un contact ponctuel reste théorique. En se déformant, les tissus entraineront un contact sur une zone de contact autour de ce point de contact théorique. On entend donc par contact « ponctuel » une zone de contact localisée au niveau d'une portion présentant un profil convexe ou courbée de l'excroissance 32. Ainsi, la portion d'arête de contact 38 de ladite au moins une excroissance 32 peut présenter un profil convexe permettant un contact théorique ponctuel avec une surface sphérique telle que la racine du muscle ciliaire.

Le profil convexe de la portion d'arête de contact 38 peut être réalisé par un bord courbé ou bien par une pluralité de bords rectilignes suivant un profil courbé.

Ladite au moins une excroissance 32 peut être configurée pour permettre au moins un contact linéaire sur la racine du muscle ciliaire 118. Ainsi, la portion d'arête de contact 38 de ladite au moins une excroissance 32 peut présenter un profil rectiligne ou concave permettant un contact linéaire avec une surface sphérique telle que la racine du muscle ciliaire.

Le profil concave de la portion d'arête de contact 38 peut être réalisé par un bord courbé ou bien par une pluralité de bords rectilignes suivant un profil courbé.

Ladite au moins une excroissance 32 s'étend le long d'un axe longitudinal A passant par la portion d'arête de contact 38.

Ladite au moins une excroissance 32 forme une saillie ou un décrochage par rapport au corps 22 permettant un contact local avec une surface convexe. Cette saillie ou ce décrochage sont de préférence séparé du reste du corps 22 d'une distance au moins égale à 0,05mm. Cette valeur de décrochage ou dimension longitudinale L est mesurée le long de l'axe longitudinal A entre les extrémités distale 40 et proximale 42 de ladite au moins excroissance 32.

Ladite au moins une excroissance 32 peut être de forme allongée. Ladite au moins une excroissance 32 présente, suivant une vue en projection dans le plan contenant la largeur et la longueur, une dimension transversale T prise au niveau de son extrémité proximale 42 selon une direction perpendiculaire à l'axe longitudinal A inférieure à sa dimension longitudinale L.

De manière alternative, ladite au moins une excroissance 32 peut être de forme aplatie, i.e. présenter, suivant une vue en projection dans le plan contenant la largeur et la longueur, une dimension transversale T prise au niveau de son extrémité proximale 42 selon une direction perpendiculaire à l'axe longitudinal A supérieure à sa dimension longitudinale L.

Les parois latérales de ladite au moins une excroissance 32 peuvent être rectilignes, courbées ou bien encore une combinaison de parois rectilignes et courbées.

Ladite au moins une excroissance 32 peut également présenter des variations de largeur, i.e. de dimension transversale T, ou une géométrie permettant à ladite au moins une excroissance 32 de se déformer élastiquement, i.e. sans déformation permanente, le long de son axe longitudinal A. En particulier, cette géométrie favorisant une déformation élastique longitudinale peut être une forme en S ou de ressort.

Plus généralement, l'arête de contact 36 définit un profil de contact 44 du bord antérieur sur la racine du muscle ciliaire. Lorsque le bord antérieur 24 comprend une pluralité d'excroissances 32, chaque portion d'arête de contact 38 s'étend suivant ce profil de contact 44.

Le profil de contact 44 s'étend de préférence le long d'une trajectoire concave de manière à améliorer le contact entre le bord antérieur 24 et la surface convexe que décrit la racine du muscle ciliaire. La concavité de la trajectoire du profil de contact 44 est considérée vis-à-vis de l'implant 20. Ainsi, on entend par trajectoire concave le fait que l'arête de contact 36 s'étendant le long de ce profil décrit une arête de contact concave capable de s'adapter au mieux à une surface convexe telle que la racine du muscle ciliaire.

Le profil de contact 44 peut notamment s'étendre le long d'une trajectoire circulaire de manière à faciliter la fabrication de l'implant, notamment lorsqu'il est réalisé par usinage. Ainsi chaque portion d'arête de contact 38 s'étend le long d'une portion de cette trajectoire circulaire.

Le profil de contact 44 peut également s'étendre le long de trajectoires différentes. Pour cela, le profil de contact 44 comprend une pluralité de portions de profil de contact 46 formant conjointement le profil de contact 44. Une portion de profil de contact 46 peut être portée par une ou plusieurs portions d'arête de contact 38, i.e. d'excroissances 32.

Pour obtenir un bon compromis entre positionnement de l'implant 20 et amélioration de la collecte d'humeur aqueuse, il est possible de former des groupes d'excroissances 32 s'étendant le long d'une même trajectoire, par exemple des paires. Le bord antérieur 24 peut ainsi présenter un premier groupe d'excroissances 32 s'étendant le long d'une première trajectoire capable de s'adapter au mieux à une première géométrie de muscle ciliaire et un deuxième groupe d'excroissances s'étendant le long d'une deuxième trajectoire capable de s'adapter au mieux à une deuxième géométrie de muscle ciliaire. Ces groupes ou paires d'excroissance 32 sont de préférence répartis le long du bord antérieur 24 pour améliorer la stabilité du contact avec la racine du muscle ciliaire.

Ces portions de profil de contact 46 peuvent notamment s'étendre le long de trajectoires différentes pour obtenir une géométrie du bord antérieur 24 capable de s'adapter à de multiples formes de surface. Ainsi, il est possible qu'une ou plusieurs portions de profil de contact 46 s'étendent le long d'une première trajectoire et qu'une ou plusieurs portions de profil de contact 46 s'étendent le long d'une deuxième trajectoire. Les trajectoires peuvent être de forme différente, de positionnement différent, d'orientation différente ou bien encore une combinaison de ces attributs différents. Le profil de contact 44 peut ainsi successivement s'étendre le long de trajectoires rectiligne et/ou concave.

Le profil de contact 44 peut notamment s'étendre le long d'au moins une trajectoire circulaire. Ainsi, le profil de contact 44 peut s'étendre le long de plusieurs trajectoires circulaires. En d'autres termes, les portions d'arêtes de contact 38 des excroissances 32 peuvent s'étendre le long de trajectoires circulaires différentes.

Selon un mode de réalisation avantageux, le profil de contact 44 peut s'étendre le long de trajectoires circulaires ayant des rayons de courbure différents. Autrement dit, les portions d'arêtes de contact 38 des excroissances 32 peuvent s'étendre le long de cercles de diamètres différents. Il est ainsi possible d'avoir des excroissances 32 dont la conformation, notamment la forme et l'orientation, sont adaptées à des surfaces convexes de rayons différents. En conséquence, le bord antérieur 24 peut s'adapter à des anatomies différentes, notamment à des yeux ayant des diamètres de chambre antérieure différents.

Ces trajectoires circulaires présentent un centre situé de préférence le long d'une même direction transversale au bord antérieur 24, suivant une vue en projection dans le plan contenant la largeur et la longueur.

Selon un mode de réalisation avantageux, le bord antérieur 24 comprend des groupes d'excroissances 32 s'étendant le long de trajectoires circulaires ayant des rayons de courbure différents. En d'autres termes, le profil de contact 44 de l'arête de contact 36 comprend au moins une première portion de profil s'étendant le long d'une première trajectoire circulaire présentant un premier rayon de courbure et au moins une deuxième portion de profil s'étendant le long d'une deuxième trajectoire circulaire présentant un deuxième rayon de courbure supérieur au premier rayon de courbure pour permettre à l'arête de contact de venir au contact de racines de muscle ciliaire ayant une pluralité de rayons anatomiques possibles.

La première portion de profil est formée au niveau d'une zone centrale de l'arête de contact. La deuxième portion de profil étant disposée au niveau d'une zone périphérique de l'arête de contact. Ainsi, la deuxième portion de profil est disposée de part et d'autre de la première portion de profil le long de l'arête de contact lorsqu'une pluralité de portions d'arête de contact, et donc d'excroissances 32, s'étendent le long de cette deuxième trajectoire.

Il a été déterminé que le diamètre de la chambre antérieure de l'œil peut varier entre 10,75mm et 13,75mm. Ainsi, le bord antérieur 24 est conformé de sorte qu'au moins un rayon de courbure de la trajectoire circulaire est supérieur ou égal à 4,5 mm et inférieur ou égal à 6 mm. De manière alternative ou combinée, le bord antérieur 24 est conformé de sorte qu'au moins un rayon de courbure de la trajectoire circulaire est supérieur à 6 mm et inférieur ou égal à 7.5 mm.

Ainsi, le bord antérieur 24 comprend au moins un groupe d'excroissances 32 s'étendant le long d'une trajectoire circulaire ayant un rayon de courbure supérieur ou égal à 4.5 mm et inférieur ou égal à 6 mm et au moins un groupe d'excroissances 32 s'étendant le long d'une trajectoire circulaire ayant un rayon de courbure supérieur à 6 mm et inférieur ou égal à 7.5 mm.

Selon une première configuration, le corps 22 de l'implant 20 n'est pas élastiquement déformable et comporte, de manière permanente, une courbure dans une direction perpendiculaire à un plan défini par les deux dimensions de l'implant 20 qui sont perpendiculaires à l'épaisseur. L'implant 20 est donc dans ce mode de réalisation un implant non déformable élastiquement et pouvant présenter une ou plusieurs courbures anatomiques. En d'autres termes, l'implant 20 peut être inséré sans déformation élastique dans son emplacement contre la racine du muscle ciliaire.

Selon une deuxième configuration, le corps 22 de l'implant 20 est élastiquement déformable de manière à pouvoir être plié sans déformation permanente pour être manipulé avec un micro-instrument, ou injecté avec un système d'injection ophtalmologique. Lorsqu'un tel corps 22 n'est plus soumis à l'effort de pliage, il reprend sa position d'origine non déformée (repos). L'implant 20 est donc dans ce mode de réalisation un implant déformable élastiquement et pouvant présenter une ou plusieurs courbures anatomiques. En d'autres termes, l'implant 20 est uniquement destiné à être placé dans un état déformé pour son insertion au travers des tissus.

Selon une troisième configuration, le corps 22 de l'implant 20 est élastiquement déformable, de manière à pouvoir être plié sans déformation permanente pour être manipulé avec un micro-instrument, ou injecté avec un système d'injection ophtalmologique. Lorsqu'un tel corps 22 n'est plus soumis à l'effort de pliage, il reste positionné in-situ dans un état déformé correspondant à l'espace anatomique entre la sclère et le tissu uvéal. En d'autres termes, l'implant 20 est destiné à être placé dans un état déformé pour son insertion au travers des tissus ainsi que durant son utilisation contre la racine du muscle ciliaire.

Lorsque positionné entre la sclère et le corps uvéal, le corps 22 de l'implant 20 est configuré pour comprendre au moins un rayon de courbure de la trajectoire circulaire qui est supérieur ou égal à 4,5 mm et inférieur ou égal à 6 mm. De manière combinée ou alternative, le corps 22 de l'implant 20 est configuré pour comprendre au moins un rayon de courbure de la trajectoire circulaire qui est supérieur à 6 mm et inférieur ou égal à 7,5 mm. Ces plages de valeur de rayons de courbure concernent chacune des trois configurations mentionnées ci-avant. Dans la troisième configuration, ces plages concernent le corps de l'implant 20 lorsqu'il est à l'état déformé et positionné entre la sclère et le corps uvéal. Ainsi, le corps de l'implant 20 est adapté à la diversité d'anatomies de l'oeil des patients lorsqu'il est positionné entre la sclère et le corps uvéal quelle que soit la configuration du corps 22.

Le corps 22 de l'implant 20 comporte, dans un état déformé dans lequel il est susceptible d'être utilisé comme implant ophtalmologique d'interposition entre la sclère et le tissu uvéal, une courbure dans une direction perpendiculaire à un plan défini par les deux dimensions de l'implant qui sont perpendiculaires à l'épaisseur. Cette courbure est de préférence concave pour s'adapter au mieux à l'anatomie du patient.

Le corps de l'implant peut être réalisé dans un matériau qui possède un module de Young compris entre 30 et 60 kg/cm2. Dans ce cas, le corps de l'implant peut comprendre au moins un matériau qui est choisi parmi les matériaux suivants : le PTFE, le polysiloxane, les hydrogels acrylates hydrophiles ou hydrophobes.

De manière alternative, le corps de l'implant peut être réalisé dans un matériau qui possède un module de Young compris entre 30 000 et 2 500 000 kg/cm2. Dans ce cas, le corps de l'implant peut comprendre au moins un matériau qui est choisi parmi les matériaux suivants : le polypropylène, le plexiglas, le titane, l'acier inoxydable, le Nitinol.

Le corps de l'implant peut présenter des propriétés de relargage d'une ou de plusieurs substances. De telles substances sont par exemple des substances anti-infectieuses et/ou anti-inflammatoires. Il peut ainsi s'agir de substances antibiotiques et/ou de substances cortisoniques ou anti-cortisoniques.

Le corps 22 de l'implant 20 peut-être, totalement ou localement, recouvert de substance(s), ou avec un état de surface judicieusement choisi, de sorte que la ou les substances, ou l'état de surface inhibent ou limitent la croissance cellulaire des tissus environnants sur l'implant 20.

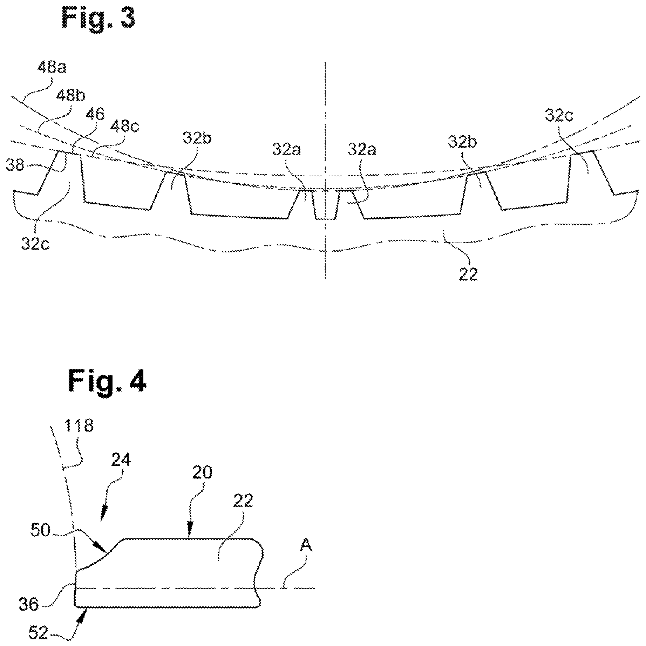

Un exemple de bord antérieur 24 comprenant des groupes d'excroissances s'étendant le long de trajectoires circulaires ayant des rayons de courbure différents est illustré en figure 3.

Le bord antérieur 24 selon cet exemple comprend trois groupes d'excroissances 32 : un premier groupe d'excroissances 32a, un deuxième groupe d'excroissances 32b et un troisième groupe d'excroissances 32c. Trois trajectoires circulaires sont représentées : une première trajectoire 48a, une deuxième trajectoire 48b et une troisième trajectoire 48c. Les rayons de courbure des trajectoires sont croissants de la première trajectoire 48a vers la troisième trajectoire 48c. Autrement dit, la troisième trajectoire 48c a un rayon de courbure supérieur à la deuxième trajectoire 48b qui a un rayon de courbure supérieur à la première trajectoire 48a.

Les portions d'arête de contact 38 du premier groupe d'excroissances 32a s'étendent le long de la première trajectoire 48a. Les portions d'arête de contact 38 du deuxième groupe d'excroissances 32b s'étendent le long de la deuxième trajectoire 48b. Les portions d'arête de contact 38 du troisième groupe d'excroissances 32c s'étendent le long de la troisième trajectoire 48c.

Dans cet exemple de la figure 3, les groupes comprennent deux excroissances 32 chacun. De manière générale, chacun des groupes d'excroissances 32 peut comprendre au moins une excroissance 32. De plus, le bord antérieur 24 peut comprendre une pluralité de groupes d'excroissances 32 s'étendant respectivement le long d'une pluralité de trajectoires circulaires de rayon de courbure différents. Il est ainsi possible de réaliser un bord antérieur capable de s'adapter à une pluralité, voir une infinité, d'anatomies différentes de la racine du muscle ciliaire.

En référence à la figure 4, l'épaisseur du bord antérieur 24 peut être amincie par rapport à l'épaisseur du corps 22. Cet amincissement peut également être réalisé sur ladite au moins une excroissance 32.

De manière préférée, cet amincissement du bord antérieur 24 est réalisé sur la portion d'extrémité du bord antérieur 24 ce qui permet de réduire la surface du bord antérieur 24 en appui sur la racine du muscle ciliaire 118 pour une épaisseur de corps 22 donnée. Ceci permet de libérer de l'espace entre la racine du muscle ciliaire 118 et le corps 22 de l'implant 20 améliorant ainsi la collecte et la circulation d'humeur aqueuse vers le corps de l'implant.

Le bord antérieur 24 comprend une portion supérieure 50 et une portion inférieure 52. L'épaisseur du bord antérieur est ainsi amincie au niveau de la portion supérieure 50. L'arête de contact 36 est portée par la portion inférieure 52 du bord antérieur 24.

Cet amincissement du bord antérieur 24 s'étend de préférence depuis l'arête de contact 36 vers le corps 22 le long de l'axe longitudinal A sur une distance inférieure ou égale à 1,5 mm.

L'amincissement est de préférence réalisé sur la totalité de la longueur du bord antérieur 24. De manière alternative, cet amincissement est réalisé sur une portion uniquement de la longueur du bord antérieur 24.

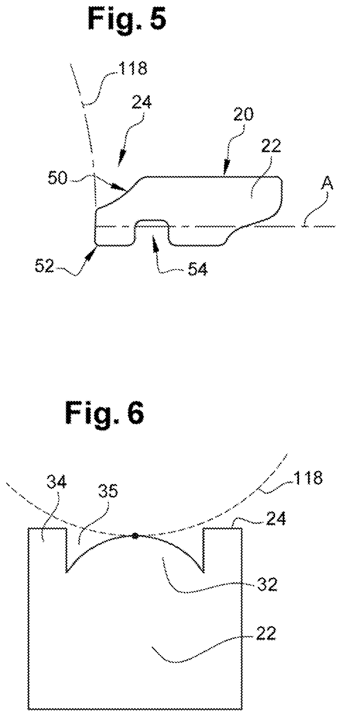

En référence à la figure 5, le bord antérieur 24 peut également comprendre un amincissement local 54 de l'épaisseur. Cet amincissement local 54 est formé sur la face supérieure ou la face inférieure de l'implant 20. Cet amincissement local 54 est configuré pour favoriser la déformation élastique du bord antérieur 24, en particulier de sa partie distale, permettant d'améliorer la surface de contact entre la racine du muscle ciliaire 118 et le bord antérieur 24. En effet, sous l'action d'un effort de plaquage de l'implant 20 sur la racine du muscle ciliaire le long de l'axe longitudinal A, l'amincissement local 54 permet d'induire une rotation de la partie distale du bord antérieur 24 vers l'amincissement local 54. Cette déformation du bord antérieur 24 favorise le contact entre le bord antérieur 24 et la racine du muscle ciliaire 118.

Le corps de l'implant peut être percé d'orifices traversant son épaisseur. Ces orifices assurent le passage d'un écoulement d'humeur aqueuse à travers le corps 22, d'une des deux grandes faces opposées à l'autre grande face opposée. Le corps peut également être percé d'une pluralité d'autres orifices situés dans d'autres plans.

Le corps 22 de l'implant 20 peut comprendre sur au moins une de ses deux grandes faces opposées un relief qui est apte à favoriser un écoulement de l'humeur aqueuse le long de ladite au moins une grande face.

Le relief peut prendre par exemple la forme de canaux ou de rainures aménagées sur les faces opposées. Les canaux ou rainures peuvent être formés en surface ou bien au travers du corps 22. Ces canaux ou rainures sont préférentiellement agencées sensiblement parallèlement à une direction qui s'étend du bord antérieur 24 du corps 22 au bord postérieur 26.

Le relief peut prendre la forme d'évidements aménagés sur ladite au moins une grande face ou d'une rugosité conférée à celle-ci.

On notera que le corps 22 de l'implant 20 peut combiner des orifices traversants, des canaux/rainures et des évidements.

Les figures 6 à 14 représentent des exemples de réalisation du corps 22 de l'implant 20 et, surtout, du bord antérieur 24.

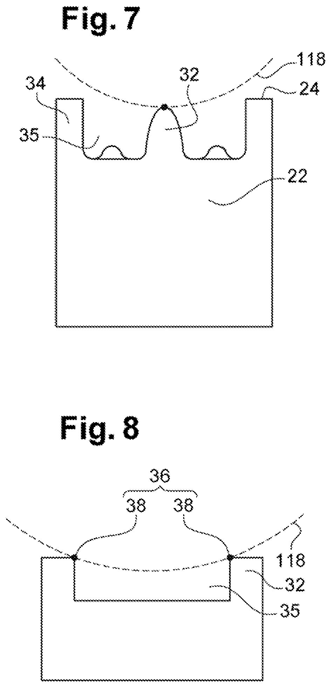

Les figures 6 et 7 représentent des exemples de bord antérieur 24 de géométries différentes comprenant une seule excroissance 32 permettant un contact local. La zone de contact local entre l'implant 20 et la racine du muscle ciliaire est symbolisée sur ces figures par un point. Comme mentionné ci-avant, la zone de contact local peut être ponctuelle ou linéaire.

Sur la figure 6, le bord antérieur 24 comprend une excroissance 32 formée par une portion centrale courbée s'étendant entre deux bras de rétention 34. Ces bras de rétention 34 conduisent l'humeur aqueuse pour favoriser leur écoulement vers le corps 22 de l'implant de manière à augmenter l'efficacité de collecte de l'humeur aqueuse.

D'une manière plus générale, le bord antérieur peut comprendre au moins un bras de rétention 34 s'étendant en saillie du corps vers l'extérieur de celui-ci. Ces bras de rétention 34 ne sont pas configurés pour entrer en contact avec la racine du muscle ciliaire 118. Ces bras de rétention 34 ont pour objectif de conduire l'humeur aqueuse pour favoriser l'écoulement de celle-ci vers le corps de l'implant 22. Le bord antérieur 24 comprend de préférence au moins deux bras de rétention 34, par exemple disposés au niveau d'extrémités latérales du bord antérieur 24.

Selon une configuration particulière, une excroissance 32 du bord antérieur 24 peut avoir une fonction de bras de rétention, notamment lorsque le bord antérieur 24 comprend au moins deux excroissances 32.

Des espaces ou évidements de collecte 35 sont formés entre l'excroissance 32 et les bras de rétention 34.

Sur la figure 7, l'excroissance 32 est formée par une protubérance allongée. L'excroissance 32 s'étend également au milieu de deux bras de rétention 34. Des évidements de collecte 35 sont également formés entre les bras de rétention 34 et l'excroissance 32.

Les exemples de géométrie du bord antérieur 24 des figures 6 et 7 comprennent des bras de rétention 34. De manière alternative, le bord antérieur 24 peut être dépourvu de bras de rétention 34.

Les figures 8 à 11 représentent des exemples de bord antérieur 24 de géométries différentes comprenant deux excroissances 32 permettant un contact local au niveau de deux zones de la racine du muscle ciliaire 118. Les zones de contact local entre l'implant 20 et la racine du muscle ciliaire 118 sont symbolisées sur ces figures par un point. Comme mentionné ci-avant, les zones de contact local peuvent être ponctuelles ou linéaires.

Sur les figures 8 à 11, les deux excroissances 32 sont formées au niveau d'une portion périphérique de ce bord antérieur 24. Autrement dit, les deux excroissances 32 sont formées au niveau d'extrémités latérales du bord antérieur 24. Un évidement de collecte 35 est formé entre les deux excroissances 32.

Sur l'ensemble de ces exemples de bord antérieur 24 des figures 8 à 11, le profil de contact 44 de l'arête de contact 36 s'étend le long d'une trajectoire concave.

Sur la figures 8, 9 et 11, les deux excroissances sont formées par une protubérance allongée. Ces deux excroissances 32 forment ici des bras de rétention d'humeur aqueuse dans l'évidement de collecte 35.

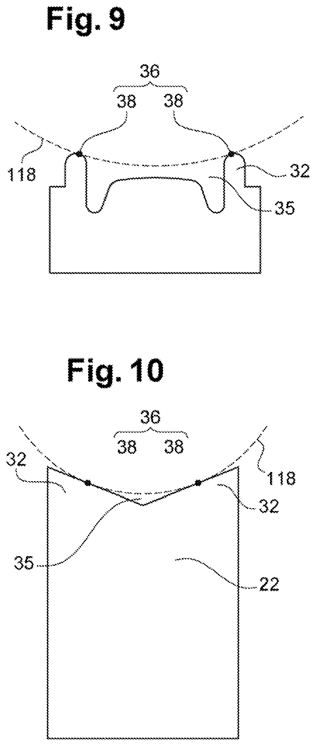

Les excroissances 32 de la figure 10 sont chacune formées par une portion rectiligne et inclinée du bord antérieur 24. Chaque portion rectiligne et inclinée se rejoint au niveau d'une portion centrale du bord antérieur 24 pour former un évidement de collecte 35.

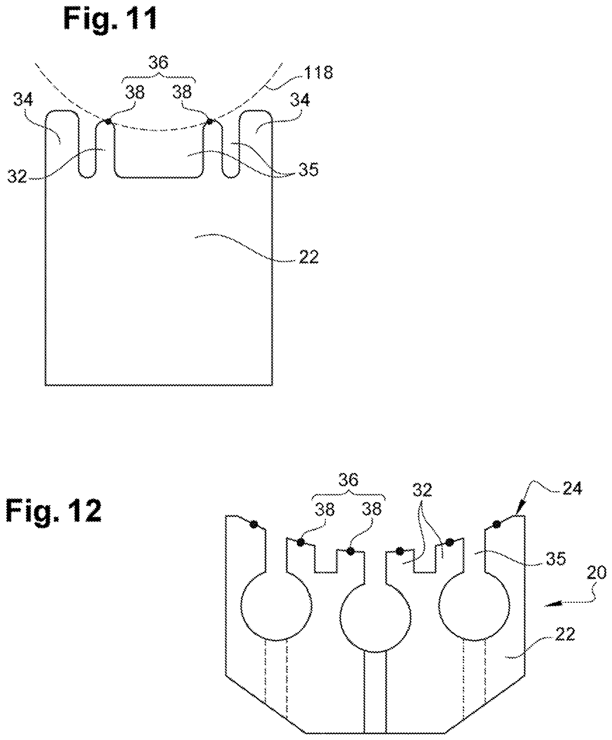

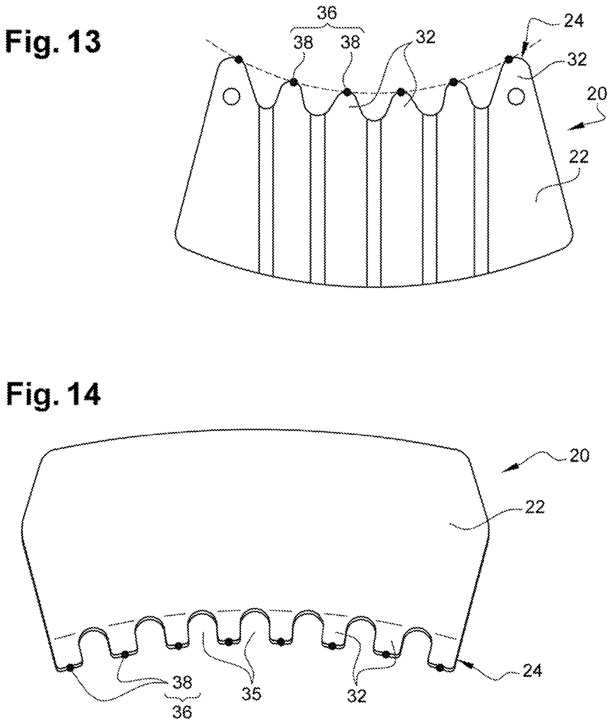

Les figures 12 et 13 représentent des exemples de bord antérieur 24 de géométries différentes comprenant une pluralité d'excroissances 32 permettant un contact local au niveau d'une pluralité de zones de la racine du muscle ciliaire 118. Les zones de contact local entre l'implant 20 et la racine du muscle ciliaire 118 sont symbolisées sur ces figures par un point.

Le profil de contact 44 de l'arête de contact 36 s'étend le long d'une trajectoire concave sur les figures 12 et 13.

Sur les figures 12 et 13, les excroissances 32 forment des portions d'arête de contact 38 toutes alignées sur une même trajectoire, en particulier circulaire.

Sur la figure 12, les excroissances 32 forment des portions d'arête de contact 38 linéaires de manière à obtenir des zones de contact linéaires avec la racine du muscle ciliaire 118.

Des évidements de collecte 35 sont formés entre les excroissances 32. Des canaux ou rainures de collecte sont formés entre ces évidements de collecte 35 et le bord postérieur 26. Ces évidements de collecte 35 ont une forme circulaire ou pseudo-circulaire.

Sur la figure 13, les excroissances 32 forment des portions d'arête de contact 38 courbées de manière à obtenir des zones de contact ponctuelles avec la racine du muscle ciliaire 118.

Des orifices de collecte sont formés aux extrémités latérales du bord antérieur 24. De manière générale, les orifices de collecte peuvent être formés tout le long du bord antérieur 24. Des canaux ou rainures de collecte s'étendent depuis le bord antérieur 24 vers le bord postérieur 26 pour permettre un écoulement d'humeur aqueuse.

La figure 14 illustre un exemple de bord antérieur 24 dont le profil de contact 44 s'étend le long d'une pluralité de trajectoires circulaires ayant des rayons de courbure différents.

De manière similaire à la figure 3, le bord antérieur de la figure 14 comprend une pluralité de groupes d'excroissances 32 dont chacune des portions d'arête de contact 38 s'étend le long d'une trajectoire différente. En particulier, l'exemple de la figure 14 comprend quatre paires d'excroissances 32 s'étendant le long de quatre trajectoires circulaires de rayons de courbure différents.

Afin d'augmenter le flux d'humeur aqueuse, un passage peut être créé au travers de la racine du muscle ciliaire pour mettre en communication directe la chambre antérieure d'un œil avec son espace supraciliaire (une cyclodialyse). Ledit passage est obtenu par une incision réalisée pendant la chirurgie de mise en place de l'implant. Néanmoins, sans précaution additionnelle cette ouverture sera rapidement refermée à cause de la cicatrisation naturelle. Elle peut être maintenue de manière plus pérenne si un objet non résorbable et non déformable la maintient ouverte.

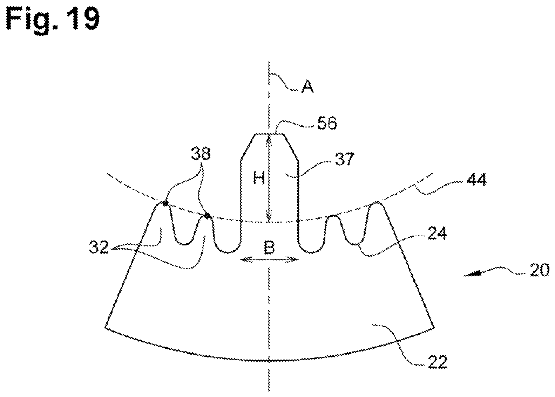

A cet effet, la figure 19 présente un mode de réalisation de l'invention où le bord antérieur 24 comprend un prolongement local faisant saillie hors du corps 20 suivant une vue en projection dans le plan contenant la largeur et la longueur. Ce prolongement local 37, nommé aiguillon, diffère d'une excroissance tel que définie plus haut en ce qu'il s'étend au-delà du profil de contact 44 et est destiné à traverser entièrement la racine du muscle ciliaire pour permettre l'établissement d'une ouverture pérenne entre la chambre antérieure et l'espace supra-ciliaire.

Le bord antérieur 24 peut présenter un ou plusieurs aiguillons 37.

Ledit au moins un aiguillon 37 s'étend le long d'un axe longitudinal A passant par la pointe distale 56.

Ledit au moins un aiguillon 37 peut être de forme allongée. Ledit aiguillon 37 présente, suivant une vue en projection dans le plan contenant la largeur et la longueur, une dimension transversale B selon une direction perpendiculaire à l'axe longitudinal A inférieure à sa dimension longitudinale H.

De manière alternative, ledit au moins un aiguillon 37 peut être de forme aplatie, i.e. présenter, suivant une vue en projection dans le plan contenant la largeur et la longueur, une dimension transversale B selon une direction perpendiculaire à l'axe longitudinal A supérieure à sa dimension longitudinale H.

Les parois latérales dudit au moins un aiguillon 37 peuvent être rectilignes, courbées ou bien encore une combinaison de parois rectilignes et courbées.

Ledit au moins un aiguillon 37 peut également présenter des variations de largeur, i.e. de dimension transversale B, ou une géométrie permettant de faciliter le passage dudit au moins un aiguillon 37 au travers du muscle ciliaire. Un telle géométrie peut être complétée par une forme distale adéquatement choisie pour empêcher le retrait dudit aiguillon de la chambre antérieure, en particulier grâce à un élargissement de la pointe distale 56 dudit au moins un aiguillon par rapport à sa base, et/ou un épaississement de ladite pointe distale.

Il a été déterminé que l'épaisseur de la racine du muscle ciliaire varie de 200 à 500 microns suivant les individus. Dans un mode de réalisation privilégié de l'invention, ledit au moins aiguillon 37 présente une dimension longitudinale H comprise entre 0,2 et 2,5 mm.

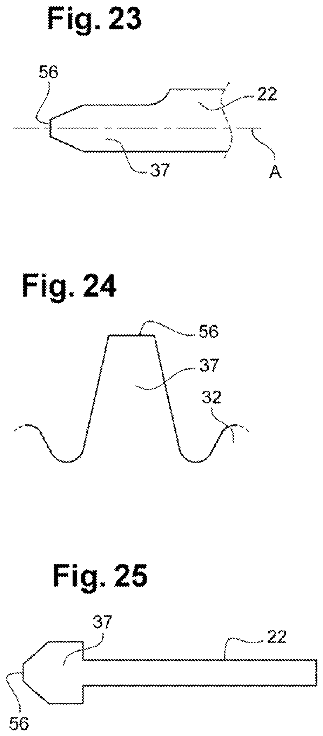

Dans un autre mode de réalisation privilégié, ledit au moins un aiguillon 37 présente une dimension transversale B comprise entre 0,2 et 2,5 mm
En référence à la figure 23, l'épaisseur de la pointe distale 56 dudit au moins un aiguillon peut être amincie par rapport à l'épaisseur du corps 22, constituant ainsi un élément facilitant la traversée du muscle ciliaire.

En référence à la figure 25, l'épaisseur de la pointe distale 56 dudit au moins un aiguillon peut être augmentée par rapport à l'épaisseur du corps 22, constituant ainsi un dispositif anti-retrait dudit au moins un aiguillon de la chambre antérieure.

La pointe distale 56 dudit au moins un aiguillon 37 peut contenir un amincissement suivi d'un épaississement, pour faciliter le passage au travers du muscle ciliaire, puis empêcher son retrait.

L'amincissement et l'épaississement peuvent être réalisés dans le plan perpendiculaire à l'épaisseur et/ou dans le plan de l'épaisseur de l'implant 20.

Ledit au moins un aiguillon 37 peut être percé d'orifices le traversant longitudinalement ou contenir des rainures ou évidements. Ces orifices, rainures ou évidements permettent le passage d'un écoulement d'humeur aqueuse à travers le muscle ciliaire

Ledit au moins un aiguillon peut être positionné soit sur le bord latéral de l'implant 20, soit entre 2 excroissance 32.

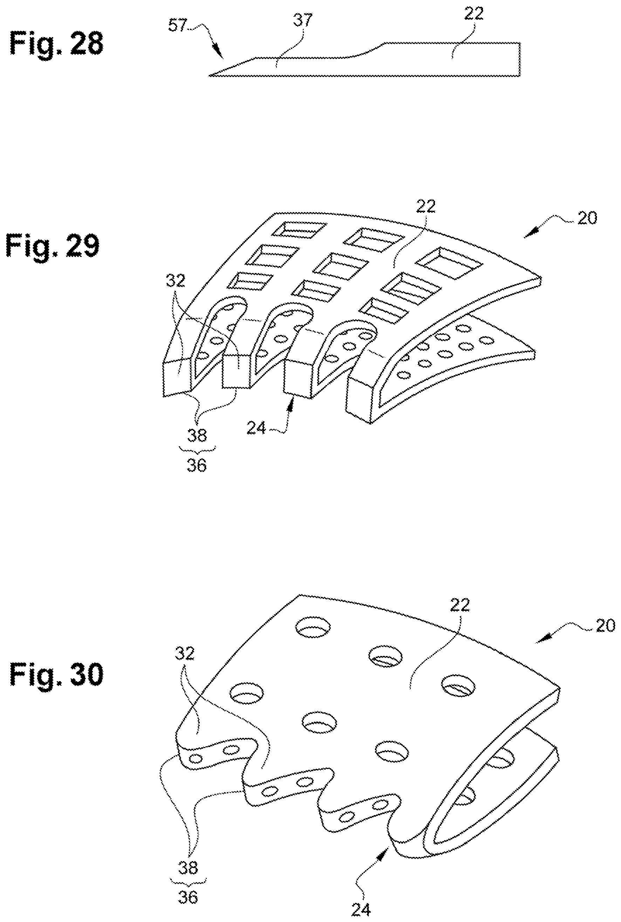

Ledit au moins un aiguillon 37 peut comprendre une pointe distale de perforation 57 configurée pour perforer une paroi de la racine du muscle ciliaire sans ouverture préalable formée dans cette paroi. Dans ce cas, l'aiguillon 37 est auto-perforant car il permet une insertion au travers des tissus sans ouverture préalable. La capacité de perforation de la pointe distale de perforation 57 peut être obtenue par un ou plusieurs bords biseautés au niveau de l'extrémité distale de l'aiguillon de sorte que la pointe distale de perforation 57 présente une section triangulaire ou triangulaire tronquée dans un plan perpendiculaire à l'épaisseur de l'implant 20. La figure 28 illustre un exemple d'aiguillon comportant une pointe distale de perforation 57.

La figure 19 illustre un exemple d'implant 20 contenant quatre excroissances 32 et un aiguillon 37 dont la distance longitudinale H est supérieure à la distance transversale B.

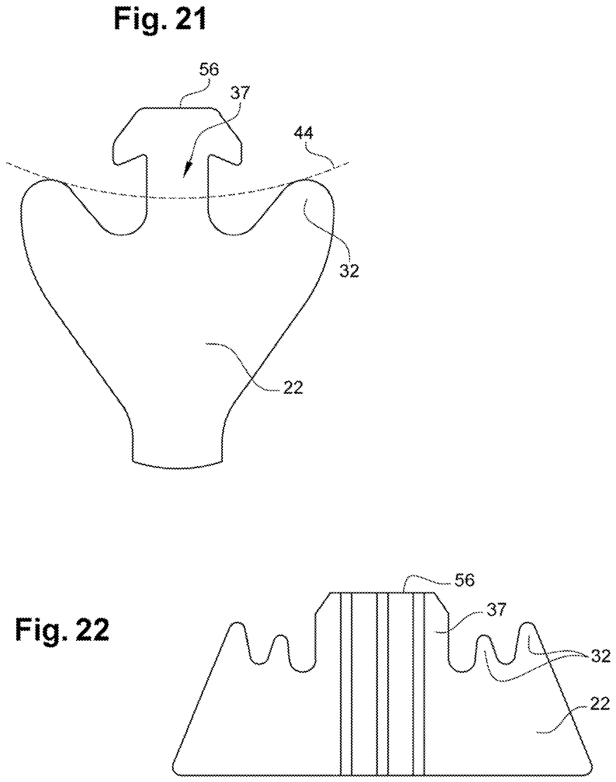

La figure 22 illustre un exemple d'implant 20 contenant quatre excroissances 32 et un aiguillon 37 dont la distance longitudinale H est inférieure à la distance transversale B. L'aiguillon comporte par ailleurs des rainures et des canaux internes.

La figure 24 illustre un exemple de géométrie dudit au moins un aiguillon permettant une insertion facilitée au travers du muscle ciliaire.

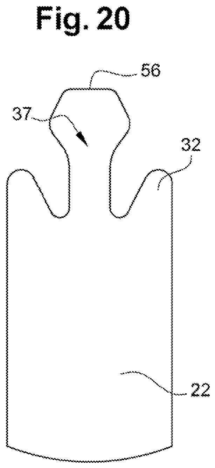

Sur la figure 20, la pointe distale du au moins un aiguillon 37 présente un premier exemple de géométrie permettant une insertion facilitée au travers du muscle ciliaire, et une résistance au retrait.

Sur la figure 21, la pointe distale dudit au moins un aiguillon 37 présente un deuxième exemple de géométrie permettant une insertion facilitée au travers du muscle ciliaire, et une résistance au retrait.

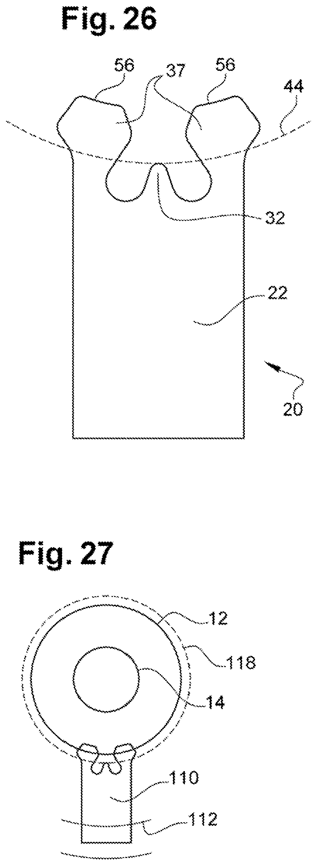

La figure 26 présente un implant 20 avec deux aiguillons 37 et une excroissance 32.

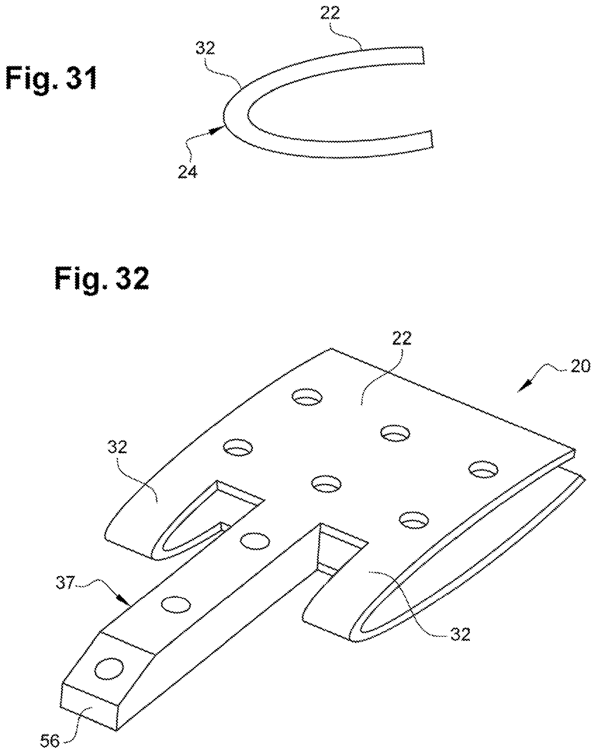

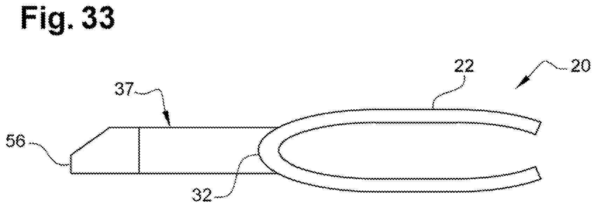

Les figures 29 à 33 représentent des exemples de réalisation de l'implant 20 sous la forme d'un corps 22 creux. Ainsi, une cavité centrale est formée entre les faces supérieure et inférieure du corps 22. Les faces supérieure et inférieure sont reliées entre elles au niveau d'un des bords du corps 22, de préférence du bord antérieur 24. Le corps 22 peut être obtenu au moyen d'une seule et même plaque repliée sur elle-même. La jonction entre les faces supérieure et inférieure au niveau du bord antérieur 24 forme également au moins une excroissance 32.

Un aiguillon 37 peut également être formé à partir du bord antérieur 24. Cet aiguillon 37 est de préférence également creux. L'aiguillon 37 comprend au moins deux parois, une paroi supérieure et une paroi inférieure.

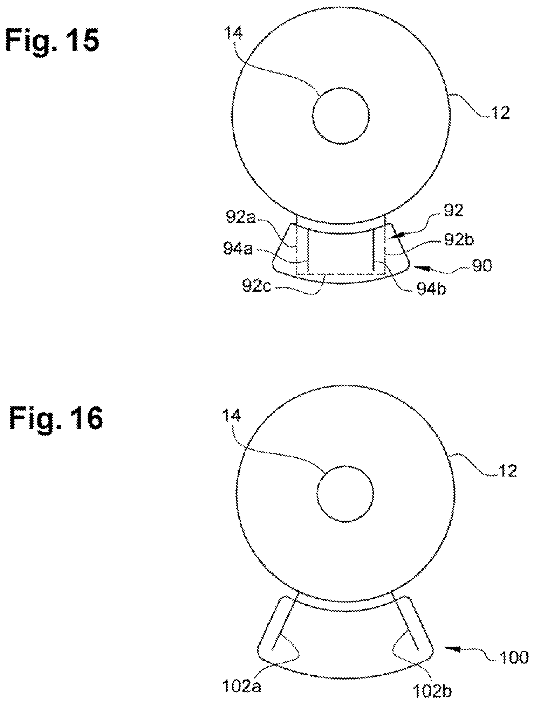

La figure 15 illustre la mise en place d'un implant 90 en utilisant une première méthode d'implantation selon un mode de réalisation de l'invention. L'implant 90 est volontairement représenté de manière simplifiée mais il est conforme à l'invention présentée ci-avant. Cette première méthode est utilisée en complément d'une intervention chirurgicale conventionnelle anti-glaucomateuse ou en complément de toute intervention intra oculaire lorsqu'un abaissement de la pression intra oculaire est souhaitable. La trabeculectomie et la sclérectomie nécessitent de découper un ou plusieurs volet(s) scléral(aux) de l'œil qui seront soulevés afin de poursuivre l'intervention. Un volet scléral 92 (illustré en pointillés sur la figure 15) est obtenu en incisant la sclère en un ou deux plans et à une profondeur variable, sur trois côtés : deux incisions 92a, 92b sensiblement parallèles entre elles qui s'étendent à partir de la cornée 12 et en éloignement de celle-ci et une troisième incision 92c perpendiculaire aux deux autres incisions et à distance de la cornée. La découpe suivant trois incisions ainsi réalisée forme ce que l'on appelle un ou plusieurs volet(s) scléral(aux). Après soulèvement du ou des volet(s) scléral(aux), deux incisions (94a, 94b sur la figure 15) sont pratiquées jusqu'au corps ciliaire, à l'intérieur du volet 92, afin de pouvoir glisser l'implant entre le plan scléral profond et le corps ciliaire. A titre d'exemple, les incisions sont espacées l'une de l'autre d'au moins 2 mm.

Selon une variante non représentée, une seule incision du plan scléral profond est réalisée pour atteindre le même but.

La méthode d'implantation utilisée peut comprendre ensuite une étape d'introduction d'une substance visco-élastique, par exemple de type acide hyaluronique, à travers au moins une des incisions réalisées, entre la sclère et le corps ciliaire afin de séparer ces deux tissus précédemment accolés. Ceci permettra la mise en place de l'implant sans traumatisme pour les structures sus-et sous-jacentes.

Cette étape est mise en œuvre par l'intermédiaire d'un instrument d'injection telle qu'une canule d'injection ayant un diamètre de l'ordre de 20 à 30g.

Une faible quantité de substance est injectée, par exemple 0,05 mm3.

Le chirurgien réalise des ouvertures entre la chambre antérieure et l'espace supraciliaire, en nombre égal au nombre d'aiguillons 37 que présente l'implant 20. Ces ouvertures sont réalisées à l'emplacement desdits aiguillons.

Le chirurgien peut s'il le désire réaliser des ouvertures supplémentaires entre la chambre antérieure et l'espace supraciliaire.

Cette ou ces ouvertures peuvent être réalisées ab-interno ou ab-externo, par exemple par action mécanique (couteau, spatule, ...) ou par laser. Pour les ouvertures supplémentaires, elles peuvent être réalisées au moment de la chirurgie ou ultérieurement.

La méthode d'implantation comprend également une étape d'introduction d'un instrument tel qu'une pince à bords mousses à travers une des deux incisions 94a, 94b qui s'étendent en profondeur jusqu'au corps ciliaire. La pince ressort par la deuxième incision et saisit l'implant pour venir le placer entre la sclère et le corps ciliaire.

Au cours d'une étape suivante, avec un instrument micro chirurgical tel qu'une spatule à bords mousses on s'assure de la position de l'implant 90 au plus près du trabeculum (de manière concentrique au limbe), comme exposé supra, afin de recueillir le maximum d'humeur aqueuse.

Un autre type d'instrument ou de dispositif permettant la mise en place, le déploiement de l'implant et son positionnement dans l'espace situé entre la sclère et le corps ciliaire (espace supra-choroïdien) peut être utilisé, tel qu'un injecteur.

Au cours d'une autre étape, le ou les volet(s) scléral(aux) sont rabattus et, suturés ou non.

La première méthode décrite s'applique aussi à la mise en place de plusieurs implants selon l'invention. De manière générale, au moins une incision différente (voire deux dans l'exemple de la figure 15) est à pratiquer pour la mise en place de chaque implant différent.

La figure 16 illustre la mise en place d'un implant 100 en utilisant une deuxième méthode d'implantation selon un mode de réalisation de l'invention. L'implant 100 est volontairement représenté de manière simplifiée mais il est conforme à l'invention présenté ci-avant.

Cette méthode est très similaire à la première méthode excepté le fait que la deuxième méthode ne vient pas en complément d'une intervention conventionnelle mais constitue une intervention en soi.

Selon cette méthode :
- deux incisions 102a, 102b préférentiellement radiales par rapport à la cornée ou préférentiellement parallèles entre elles (comme les incisions 94a, 94b de la figure 15) sont pratiquées, l'extrémité antérieure des incisions se situant entre 0 et 3 mm postérieure au limbe (zone de transition entre la cornée et la sclère ), et d'une longueur allant par exemple de 1 à 4 mm, et poursuivies jusqu'au corps ciliaire,
- une substance visco-élastique, par exemple de type acide hyaluronique, peut-être injectée à travers l'une des deux incisions,
- le cas échéant suivant le besoin ou le désir du chirurgien, des ouvertures entre la chambre antérieure et l'espace supra-ciliaire sont réalisées suivant les étapes décrites pour la première méthode,
- la sclère est soulevée afin de permettre l'insertion et la mise en place de l'implant 100. Les étapes d'insertion sont identiques à celles décrites précédemment pour la première méthode,
- l'étape finale de suture des incisions est toujours optionnelle.

Selon une variante de réalisation non représentée, une seule incision est pratiquée au cours de cette deuxième méthode et suffit pour venir installer en position d'interposition un implant entre la sclère et le corps ciliaire.

La figure 27 illustre la mise en place d'un implant 110 en utilisant une troisième méthode d'implantation selon un mode de réalisation de l'invention. L'implant 110 est volontairement représenté de manière simplifiée mais il est conforme à l'invention présentée ci-avant.

La deuxième méthode décrite s'applique aussi à la mise en place de plusieurs implants selon l'invention. De manière générale, au moins une incision différente (voire deux dans l'exemple de la figure 27) est à pratiquer pour la mise en place de chaque implant différent.

Cette méthode peut être réalisée en complément d'une chirurgie filtrante ordinaire, ou indépendamment de tout autre acte interventionnel.

Selon cette méthode : une incision 112 préférentiellement concentrique au limbe est réalisée entre 1 à 5 mm du limbe, sur une distance de 0.5 à 4 mm par exemple.

Une substance visco-élastique, par exemple de type acide hyaluronique, peut-être injectée à travers cette incision.

Le chirurgien réalise des ouvertures entre la chambre antérieure et l'espace supra-ciliaire, en nombre égal au nombre d'aiguillons 37 que présente l'implant 20. Ces ouvertures sont réalisées à l'emplacement desdits aiguillons. Le chirurgien peut s'il le désire réaliser des ouvertures supplémentaires entre la chambre antérieure et l'espace supra-ciliaire.

Cette ou ces ouvertures peuvent être réalisées ab-interno ou ab-externo, par exemple par action mécanique (couteau, spatule, ...) ou par laser. Pour les ouvertures supplémentaires, elles peuvent être réalisées au moment de la chirurgie ou ultérieurement.

L'implant est alors inséré à l'aide d'une pince sous la sclère suivant un axe perpendiculaire au limbe, en direction de la chambre antérieure, jusqu'au contact des excroissances de l'implant avec la racine du muscle ciliaire 118. Pour les implants 110 présentant un ou plusieurs aiguillons 37, cela implique que les aiguillons passent par les ouvertures réalisées par le chirurgien et sont alors présents en chambre antérieure.

Un autre type d'instrument ou de dispositif permettant la mise en place, le déploiement de l'implant et son positionnement dans l'espace situé entre la sclère et le corps ciliaire (espace supra-choroidien) peut être utilisé, tel qu'un injecteur.

Si la totalité de l'implant n'est pas insérée sous la sclère, la partie postérieure restante de l'implant est enfouie sous la sclère en direction opposée à la chambre antérieure.

L'étape finale de suture des incisions est toujours optionnelle.

Dans le mode de réalisation où l'aiguillon 37 comporte une pointe distale de perforation 57, le chirurgien peut s'affranchir de pratiquer une ou plusieurs incisions entre la chambre antérieure et l'espace supra-ciliaire comme décrit dans les 3 méthodes ci-avant. Dans ce cas, l'étape de positionnement de l'implant 20 contre la racine du muscle ciliaire comprend la perforation par la pointe distale de perforation 57 de la racine du muscle ciliaire pour insérer l'aiguillon 37 à l'intérieur de la chambre antérieure jusqu'à ce que ladite au moins une excroissance 32 soit au contact de la racine du muscle ciliaire.

On notera que les implants 90, 100 et 110 représentés sur les figures 15, 16 et 27 peuvent être l'un quelconque des implants décrits ci-dessus. Les méthodes de mise en place d'implants décrites ci-dessus s'appliquent à tout implant selon l'invention et notamment à un implant ophtalmologique d'interposition permanente entre la sclère et le tissu uvéal qui comprend un corps mince uvéocompatible formé d'une seule partie, le corps de l'implant comprenant deux bords opposés qui sont éloignés l'un de l'autre suivant une des deux dimensions perpendiculaires à l'épaisseur. L'implant peut en outre comporter l'une quelconque (ou plusieurs, voire toutes) des caractéristiques présentées dans la description générale ainsi que dans les différents modes de réalisation et variantes.

La troisième méthode décrite s'applique aussi à la mise en place de plusieurs implants selon l'invention. De manière générale, au moins une incision différente est à pratiquer pour la mise en place de chaque implant différent.

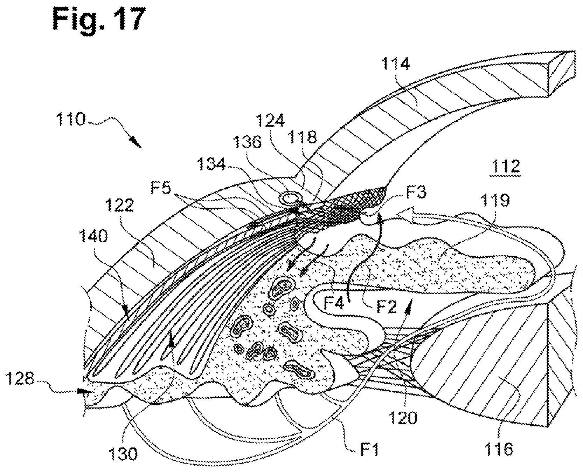

La figure 17 représente, en coupe, un implant selon un mode de réalisation de l'invention qui a été mis en place suivant l'une des méthodes décrites ci-dessus.

Cette coupe d'une partie d'un œil 110 représente la chambre antérieure 112 qui est disposée entre la cornée 114 et le cristallin 116 délimité à sa partie périphérique par l'iris 119.

Derrière l'iris 119 est disposée la chambre postérieure 120.

La sclère 122 est raccordée à la périphérie de la cornée 114 via le limbe 124 (zone de changement du rayon de courbure entre la sclère et la cornée). La sclère 122 recouvre le corps ciliaire 128 qui est raccordé à l'iris 119 et qui comprend le muscle ciliaire 130 sur lequel s'appuie la sclère 122.

Le trabeculum 134 disposé entre la cornée et l'iris fait office de filtre et est traversé par l'humeur aqueuse qui circule dans la chambre antérieure 112.

Le canal de Schlemm 136 est situé entre la sclère et la cornée en arrière du trabeculum 134.

Les différentes flèches F1, F2, F3 et F4 illustrent les chemins ou trajets empruntés par l'humeur aqueuse :
- F1 représente le chemin ou flux conventionnel emprunté par l'humeur aqueuse entrant dans la chambre antérieure 112 ;
- F2 représente le chemin ou flux de diffusion emprunté par l'humeur aqueuse pour entrer dans la chambre antérieure 112 ;
- F3 représente le chemin ou flux conventionnel emprunté par l'humeur aqueuse sortant de la chambre antérieure 112 à travers le trabeculum 134 et se dirigeant vers le canal de Schlemm 136 ;
- F4 représente l'écoulement physiologique uvéo-scléral conventionnel de l'humeur aqueuse sortant de la chambre antérieure 112.

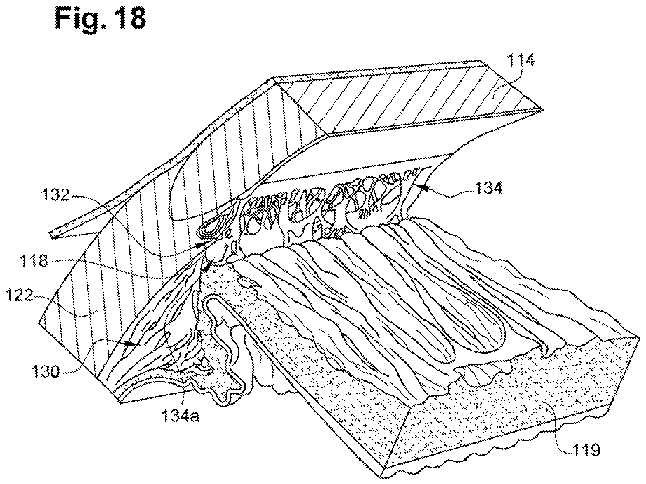

Un implant 140 selon un mode de réalisation de l'invention a été interposé entre la sclère 122 et le muscle ciliaire 130 comme décrit ci-dessus. Cet implant est positionné le plus près possible de la racine du muscle ciliaire 118(grâce à son bord antérieur concave) afin d'exercer son effet d'écartement permanent à l'endroit le plus approprié, tout en respectant l'insertion du muscle ciliaire 130 à l'éperon scléral. La figure 18 est une vue plus détaillée et agrandie de la structure de l'angle iridocornéen sans l'implant. Comme représenté sur cette figure, l'éperon scléral 132 sur lequel est inséré le muscle ciliaire 130 est situé au-dessus de la partie postérieure 134a du trabeculum 134.

L'écartement produit à cet endroit entre la sclère et le corps ciliaire permet de recueillir de manière permanente l'humeur aqueuse au plus près de la zone de l'écoulement physiologique uvéo-scléral (l'effet d'écartement crée une zone de moindre résistance à l'écoulement de l'humeur aqueuse). Un tel implant ainsi positionné apporte un gain significatif dans l'augmentation de l'écoulement uvéo-scléral physiologique.

L'écoulement physiologique uvéo-scléral est accru par une fraction supplémentaire d'écoulement à travers la partie postérieure du trabeculum (trabeculum ciliaire), comme représenté sur la figure 17 par les flèches F5 situées au-dessus et en dessous de l'implant. L'écoulement de cette fraction supplémentaire est obtenu grâce à l'effet d'écartement de l'implant entre la sclère et le corps ciliaire, au plus près de la racine du muscle ciliaire 118, sans toutefois endommager cette dernière.

## Revendications

1. Implant (20) ophtalmologique d'interposition permanente entre la sclère et le tissu uvéal, comprenant un corps (22) uvéocompatible, le corps comportant trois dimensions dans l'espace, à savoir une longueur et une largeur qui sont perpendiculaires entre elles et à une épaisseur, le corps de l'implant comprenant un premier bord dit antérieur (24) destiné à être orienté en direction de la chambre antérieure d'un œil au contact de la racine du muscle ciliaire et un deuxième bord dit postérieur (26) opposé au bord antérieur (24) par rapport au corps (22), **caractérisé en ce que** le bord antérieur présente suivant une vue en projection dans le plan contenant la largeur et la longueur, au moins une excroissance (32) orientée vers l'extérieur du corps (22) pour permettre au moins un contact local de ladite au moins une excroissance (32) sur la racine du muscle ciliaire (118).

2. Implant (20) ophtalmologique d'interposition selon la revendication 1, dans lequel le bord antérieur (24) comprend une pluralité d'excroissances (32) orientées vers l'extérieur du corps (22), lesdites excroissances formant conjointement une arête de contact (36) discontinu ou local sur la racine du muscle ciliaire (118).

3. Implant (20) ophtalmologique d'interposition selon la revendication 2, dans lequel l'arête de contact (36) définit un profil de contact (44) du bord antérieur (24) sur la racine du muscle ciliaire (118), ce profil de contact (44) s'étendant le long d'une trajectoire concave.

4. Implant (20) ophtalmologique d'interposition selon la revendication 3, dans lequel le profil de contact (44) de l'arête de contact (36) s'étend le long d'au moins une trajectoire circulaire (48a, 48b, 48c).

5. Implant (20) ophtalmologique d'interposition selon la revendication 3 ou 4, dans lequel le profil de contact (44) de l'arête de contact (36) comprend au moins une portion de profil (46) s'étendant le long d'une trajectoire circulaire (48a, 48b, 48c).

6. Implant (20) ophtalmologique d'interposition selon la revendication 5, dans lequel dans lequel le profil de contact (44) de l'arête de contact (36) comprend au moins deux portions de profil (46) s'étendant le long de trajectoires circulaires (48a, 48b, 48c) ayant des rayons de courbure différents.

7. Implant (20) ophtalmologique d'interposition selon la revendication 6, dans lequel le profil de contact (44) de l'arête de contact (36) comprend au moins une première portion de profil s'étendant le long d'une première trajectoire circulaire présentant un premier rayon de courbure et au moins une deuxième portion de profil s'étendant le long d'une deuxième trajectoire circulaire présentant un deuxième rayon de courbure supérieur au premier rayon de courbure pour permettre à l'arête de contact (36) de venir au contact de racine de muscle ciliaire ayant une pluralité de rayons anatomiques possibles.

8. Implant (20) ophtalmologique d'interposition selon la revendication 7, dans lequel la première portion de profil est formée au niveau d'une zone centrale de l'arête de contact, la deuxième portion de profil étant disposée au niveau d'une zone périphérique de l'arête de contact.

9. Implant (20) ophtalmologique d'interposition selon la revendication 7 ou 8, dans lequel la deuxième portion de profil est disposée de part et d'autre de la première portion de profil le long de l'arête de contact (36).

10. Implant (20) ophtalmologique d'interposition selon l'une quelconque des revendications 4 à 9, dans lequel au moins un rayon de courbure de la trajectoire circulaire (48a, 48b, 48c) est supérieur ou égal à 4,5 mm et inférieur ou égal à 6 mm.

11. Implant (20) ophtalmologique d'interposition selon l'une quelconque des revendications 4 à 10, dans lequel au moins un rayon de courbure de la trajectoire circulaire (48a, 48b, 48c) est supérieur à 6 mm et inférieur ou égal à 7,5 mm.

12. Implant (20) ophtalmologique d'interposition selon l'une quelconque des revendications précédentes, dans lequel le bord antérieur (24) comprend au moins un aiguillon (37) s'étendant vers l'extérieur du corps (22) et faisant saillie au-delà de ladite au moins une excroissance (32) de manière à pouvoir traverser une paroi de la racine du muscle ciliaire (118) lorsque ladite au moins excroissance (32) est au contact de la racine du muscle ciliaire (118).

13. Implant (20) ophtalmologique d'interposition selon la revendication 12, dans lequel l'aiguillon (37) comprend une pointe distale de perforation (57) configurée pour perforer une paroi de la racine du muscle ciliaire (118) sans ouverture préalable formée dans cette paroi.

14. Implant (20) ophtalmologique d'interposition selon la revendication 12 ou 13, dans lequel ledit au moins un aiguillon (37) comprend un canal de collecte de l'humeur aqueuse s'étendant depuis une extrémité dudit au moins aiguillon (37) vers le corps (22) de l'implant.

15. Implant (20) ophtalmologique d'interposition selon l'une quelconque des revendications précédentes, dans lequel le corps (22) forme au moins un évidemment de collecte de l'humeur aqueuse débouchant au niveau du bord antérieur (24).

16. Implant (20) ophtalmologique d'interposition selon la revendication 15 en combinaison avec la revendication 2, dans lequel ledit au moins un évidemment est formé entre deux excroissances (32).

17. Implant (20) ophtalmologique d'interposition selon l'une des revendications précédentes, dans lequel le corps (22) de l'implant est réalisé dans un matériau qui possède un module de Young compris entre 30 et 60 kg/cm2 ou bien entre 30 000 et 2 500 000 kg/cm2.

18. Implant (20) ophtalmologique d'interposition selon l'une quelconque des revendications 1 à 15, dans lequel le corps (22) de l'implant n'est pas élastiquement déformable et comporte, de manière permanente, une courbure dans une direction perpendiculaire à un plan défini par les deux dimensions de l'implant qui sont perpendiculaires à l'épaisseur, le corps (22) de l'implant possédant deux grandes faces opposées écartées l'une de l'autre suivant l'épaisseur du corps, les deux grandes faces opposées comprenant une face supérieure et une face inférieure séparées par l'épaisseur, le corps de l'implant ayant l'une des conformations suivantes dans une direction perpendiculaire à un plan défini par les deux dimensions de l'implant qui sont perpendiculaires à l'épaisseur :
- la face supérieure est plane et la face inférieure est concave,
- la face supérieure est plane et la face inférieure est convexe,
- les faces supérieure et inférieure sont convexes,
- les faces supérieure et inférieure sont planes.

## Patentansprüche

1. Ophthalmologisches Implantat (20) zur dauerhaften Zwischenpositionierung zwischen der Sklera und dem Uvealgewebe, das einen uveal kompatiblen Körper (22) umfasst, wobei der Körper drei Dimensionen im Raum umfasst, und zwar eine Länge und eine Breite, die senkrecht zueinander und zu einer Dicke sind, wobei der Körper des Implantats einen ersten sogenannten vorderen Rand (24), der dazu bestimmt ist, in Richtung der vorderen Kammer eines Auges in Kontakt mit der Wurzel des Ziliarmuskels ausgerichtet zu werden, und einen zweiten sogenannten hinteren Rand (26), der zu dem vorderen Rand (24) in Bezug auf den Körper (22) entgegengesetzt ist, umfasst, **dadurch gekennzeichnet, dass** der vordere Rand in einer Projektionsansicht in der die Breite und die Länge enthaltenden Ebene mindestens einen Vorsprung (32) aufweist, der nach außerhalb des Körpers (22) ausgerichtet ist, um mindestens einen lokalen Kontakt des mindestens einen Vorsprungs (32) an der Wurzel des Ziliarmuskels (118) zu ermöglichen.

2. Ophthalmologisches Implantat (20) zur Zwischenpositionierung nach Anspruch 1, wobei der vordere Rand (24) eine Mehrzahl von Vorsprüngen (32) umfasst, die nach außerhalb des Körpers (22) ausgerichtet sind, wobei die Vorsprünge zusammen eine diskontinuierliche oder lokale Kontaktkante (36) an der Wurzel des Ziliarmuskels (118) bilden.

3. Ophthalmologisches Implantat (20) zur Zwischenpositionierung nach Anspruch 2, wobei die Kontaktkante (36) ein Kontaktprofil (44) des vorderen Rands (24) an der Wurzel des Ziliarmuskels (118) definiert, wobei sich dieses Kontaktprofil (44) entlang einer konkaven Bahn erstreckt.

4. Ophthalmologisches Implantat (20) zur Zwischenpositionierung nach Anspruch 3, wobei sich das Kontaktprofil (44) der Kontaktkante (36) entlang mindestens einer Kreisbahn (48a, 48b, 48c) erstreckt.

5. Ophthalmologisches Implantat (20) zur Zwischenpositionierung nach Anspruch 3 oder 4, wobei das Kontaktprofil (44) der Kontaktkante (36) mindestens einen Profilabschnitt (46) umfasst, der sich entlang einer Kreisbahn (48a, 48b, 48c) erstreckt.

6. Ophthalmologisches Implantat (20) zur Zwischenpositionierung nach Anspruch 5, wobei das Kontaktprofil (44) der Kontaktkante (36) mindestens zwei Profilabschnitte (46) umfasst, die sich entlang von Kreisbahnen (48a, 48b, 48c) mit unterschiedlichen Krümmungsradien erstrecken.

7. Ophthalmologisches Implantat (20) zur Zwischenpositionierung nach Anspruch 6, wobei das Kontaktprofil (44) der Kontaktkante (36) mindestens einen ersten Profilabschnitt, der sich entlang einer ersten Kreisbahn erstreckt, die einen ersten Krümmungsradius aufweist, und mindestens einen zweiten Profilabschnitt, der sich entlang einer zweiten Kreisbahn erstreckt, die einen zweiten Krümmungsradius aufweist, der größer als der erste Krümmungsradius ist, umfasst, um der Kontaktkante (36) zu ermöglichen, in Kontakt mit der Wurzel des Ziliarmuskels zu gelangen, der eine Mehrzahl von möglichen anatomischen Radien hat.

8. Ophthalmologisches Implantat (20) zur Zwischenpositionierung nach Anspruch 7, wobei der erste Profilabschnitt an einem zentralen Bereich der Kontaktkante gebildet ist, wobei der zweite Profilabschnitt an einem peripheren Bereich der Kontaktkante angeordnet ist.

9. Ophthalmologisches Implantat (20) zur Zwischenpositionierung nach Anspruch 7 oder 8, wobei der zweite Profilabschnitt beidseits des ersten Profilabschnitts entlang der Kontaktkante (36) angeordnet ist.

10. Ophthalmologisches Implantat (20) zur Zwischenpositionierung nach einem der Ansprüche 4 bis 9, wobei mindestens ein Krümmungsradius der Kreisbahn (48a, 48b, 48c) größer oder gleich 4,5 mm und kleiner oder gleich 6 mm ist.

11. Ophthalmologisches Implantat (20) zur Zwischenpositionierung nach einem der Ansprüche 4 bis 10, wobei mindestens ein Krümmungsradius der Kreisbahn (48a, 48b, 48c) größer als 6 mm und kleiner oder gleich 7,5 mm ist.

12. Ophthalmologisches Implantat (20) zur Zwischenpositionierung nach einem der vorhergehenden Ansprüche, wobei der vordere Rand (24) mindestens einen Dorn (37) umfasst, der sich nach außerhalb des Körpers (22) erstreckt und über den mindestens einen Vorsprung (32) hinausragt, so dass er eine Wand der Wurzel des Ziliarmuskels (118) durchdringen kann, wenn der mindestens eine Vorsprung (32) in Kontakt mit der Wurzel des Ziliarmuskels (118) ist.

13. Ophthalmologisches Implantat (20) zur Zwischenpositionierung nach Anspruch 12, wobei der Dorn (37) eine distale Durchstechspitze (57) umfasst, die dazu ausgebildet ist, eine Wand der Ziliarmuskelwurzel (118) ohne eine in dieser Wand gebildete vorherige Öffnung zu durchstechen.

14. Ophthalmologisches Implantat (20) zur Zwischenpositionierung nach Anspruch 12 oder 13, wobei der mindestens eine Dorn (37) einen Kanal zum Sammeln des Kammerwassers umfasst, der sich von einem Ende des mindestens einen Dorns (37) zu dem Körper (22) des Implantats hin erstreckt.

15. Ophthalmologisches Implantat (20) zur Zwischenpositionierung nach einem der vorhergehenden Ansprüche, wobei der Körper (22) mindestens eine Ausnehmung zum Sammeln des Kammerwassers bildet, die an dem vorderen Rand (24) mündet.

16. Ophthalmologisches Implantat (20) zur Zwischenpositionierung nach Anspruch 15 in Kombination mit Anspruch 2, wobei die mindestens eine Ausnehmung zwischen zwei Vorsprüngen (32) gebildet ist.

17. Ophthalmologisches Implantat (20) zur Zwischenpositionierung nach einem der vorhergehenden Ansprüche, wobei der Körper (22) des Implantats aus einem Material ausgeführt ist, das einen Elastizitätsmodul zwischen 30 und 60 kg/cm2 oder aber zwischen 30.000 und 2.500.000 kg/cm2 aufweist.

18. Ophthalmologisches Implantat (20) zur Zwischenpositionierung nach einem der Ansprüche 1 bis 15, wobei der Körper (22) des Implantats nicht elastisch verformbar ist und dauerhaft eine Krümmung in eine Richtung aufweist, die senkrecht zu einer Ebene ist, die durch die beiden Dimensionen des Implantats definiert wird, die senkrecht zu der Dicke verlaufen, wobei der Körper (22) des Implantats zwei entgegengesetzte Hauptflächen besitzt, die entlang der Dicke des Körpers voneinander beabstandet sind, wobei die entgegengesetzten Hauptflächen eine obere Fläche und eine untere Fläche umfassen, die durch die Dicke getrennt sind, wobei der Körper des Implantats eine der folgenden Ausgestaltungen in einer Richtung senkrecht zu einer Ebene hat, die durch die beiden Dimensionen des Implantats definiert wird, die senkrecht zu der Dicke verlaufen:
- die obere Fläche ist eben und die untere Fläche ist konkav,
- die obere Fläche ist eben und die untere Fläche ist konvex,
- die obere und die untere Fläche sind konvex,
- die obere und die untere Fläche sind eben.

## Claims

1. Ophthalmological implant (20) for permanent interposition between the sclera and the uveal tissue, comprising a uveo-compatible body (22), the body having three dimensions in space, namely a length and a width that are perpendicular to each other and to a thickness, the body of the implant comprising a first or anterior edge (24) intended to be oriented in the direction of the anterior chamber of an eye in contact with the root of the ciliary muscle and a second or posterior edge (26) opposite the anterior edge (24) relative to the body (22), **characterized in that** the anterior edge has, as seen in a view in projection in the plane containing the width and the length, at least one protuberance (32) oriented toward the exterior of the body (22) in order to enable at least one local contact of said at least one protuberance (32) on the root of the ciliary muscle (118).

2. Interpositional ophthalmological implant (20) according to Claim 1 in which the anterior edge (24) comprises a plurality of protuberances (32) oriented toward the exterior of the body (22), said protuberances conjointly forming a discontinuous or local contact edge (36) on the root of the ciliary muscle (118).

3. Interpositional ophthalmological implant (20) according to Claim 2 in which the contact edge (36) defines a contact profile (44) of the anterior edge (24) on the root of the ciliary muscle (118), said contact profile (44) extending along a concave trajectory.

4. Interpositional ophthalmological implant (20) according to Claim 3 in which the contact profile (44) of the contact edge (36) extends along at least one circular trajectory (48a, 48b, 48c).

5. Interpositional ophthalmological implant (20) according to Claim 3 or 4 in which the contact profile (44) of the contact edge (36) comprises at least one profile portion (46) extending along a circular trajectory (48a, 48b, 48c).

6. Interpositional ophthalmological implant (20) according to Claim 5 in which the contact profile (44) of the contact edge (36) comprises at least two profile portions (46) extending along circular trajectories (48a, 48b, 48c) having different radii of curvature.

7. Interpositional ophthalmological implant (20) according to Claim 6 in which the contact profile (44) of the contact edge (36) comprises at least one first profile portion extending along a first circular trajectory having a first radius of curvature and at least one second profile portion extending along a second circular trajectory having a second radius of curvature greater than the first radius of curvature to enable the contact edge (36) to come into contact with the root of the ciliary muscle having a plurality of possible anatomical radii.

8. Interpositional ophthalmological implant (20) according to Claim 7 in which the first profile portion is formed at the level of a central zone of the contact edge, the second profile portion being disposed at the level of a peripheral zone of the contact edge.

9. Interpositional ophthalmological implant (20) according to Claim 7 or 8 in which the second profile portion is disposed on either side of the first profile portion along the contact edge (36).

10. Interpositional ophthalmological implant (20) according to any one of Claims 4 to 9 in which at least one radius of curvature of the circular trajectory (48a, 48b, 48c) is greater than or equal to 4.5 mm and less than or equal to 6 mm.

11. Interpositional ophthalmological implant (20) according to any one of Claims 4 to 10 in which at least one radius of curvature of the circular trajectory (48a, 48b, 48c) is greater than 6 mm and less than or equal to 7.5 mm.

12. Interpositional ophthalmological implant (20) according to any one of the preceding claims in which the anterior edge (24) comprises at least one needle (37) extending toward the exterior of the body (22) and projecting beyond said at least one protuberance (32) in such a manner as to be able to pass through a wall of the root of the ciliary muscle (118) when said at least one protuberance (32) is in contact with the root of the ciliary muscle (118).

13. Interpositional ophthalmological implant (20) according to Claim 12, in which the needle (37) comprises a distal perforation point (57) configured to perforate a wall of the root of the ciliary muscle (118) without an opening being formed beforehand in said wall.

14. Interpositional ophthalmological implant (20) according to Claim 12 or 13 in which said at least one needle (37) comprises an aqueous humour collecting channel extending from one end of said at least one needle (37) to the body (22) of the implant.

15. Interpositional ophthalmological implant (20) according to any one of the preceding claims, in which the body (22) forms at least one aqueous humour collecting recess opening at the level of the anterior edge (24).

16. Interpositional ophthalmological implant (20) according to Claim 15 in combination with Claim 2 in which said at least one recess is formed between two protuberances (32).

17. Interpositional ophthalmological implant (20) according to any one of the preceding claims, in which the body (22) of the implant is made of a material that has a Young's modulus of between 30 and 60 kg/cm2 or between 30 000 and 2 500 000 kg/cm2.

18. Interpositional ophthalmological implant (20) according to any one of Claims 1 to 15, in which the implant body (22) is not elastically deformable and permanently has a curvature in a direction perpendicular to a plane defined by the two dimensions of the implant that are perpendicular to the thickness, the implant body (22) having two opposing major faces spaced apart from each other along the thickness of the body, the two opposing major faces comprising an upper face and a lower face separated by the thickness, the implant body having one of the following conformations in a direction perpendicular to a plane defined by the two dimensions of the implant that are perpendicular to the thickness:
- the upper face is plane and the lower face is concave,
- the upper face is plane and the lower face is convex,
- the upper and lower faces are convex,
- the upper and lower faces are plane.
